# EUROPEAN PATENT APPLICATION

(11) **EP 1 837 331 A2**
(43) Date of publication of application: **26.09.2007**
(21) Application number: 07005838.3
(22) Date of filing: 21.03.2007
(51) Int. Cl.: C07D 215/227

(54) **New crystalline aripiprazole salts and processes for preparation and purification thereof**

(30) Priority: 21.03.2006 US 385412
(71) Applicant: CHEMAGIS LTD., 51200 Bnei Brak (IL)
(72) Inventor: Brand, Michael, Ra'anana 43252 (IL); Shookrun, Moti, Petach Tikva 49507 (IL); Gribun, Irina, Bat Yam 59200 (IL); Adin, Itai, Beer Sheva 84684 (IL); Iustain, Carmen, Beer Sheva 84750 (IL); Arad, Oded, Rehovot 76303 (IL); Kaspi, Joseph, Givatayim 53201 (IL)
(74) Representative: Albrecht, Thomas

(57) **Abstract**

Provided are novel crystalline carboxylic acid salts of aripiprazole [7-(4-(4-(2,3-dichlorophenyl)-1-piperazinyl)-butoxy)-3,4-dihydro-2(1*H*)-quinolinone], methods of using such salts, and processes for producing such salts.

## Description

### BACKGROUND OF THE INVENTION

Aripiprazole (1), also known by its chemical names 7-(4-(4-(2,3-dichorophenyl)-1-piperazinyl)-butoxy)-3,4-dihydro-2(1*H*)-carbostyril or 7-(4-(4-(2,3-dichlorophenyl)-1-piperazinyl)-butoxy)-3,4-dihydro-2(1*H*)-quinolinone, is an atypical antipsychotic agent useful for the treatment of schizophrenia.

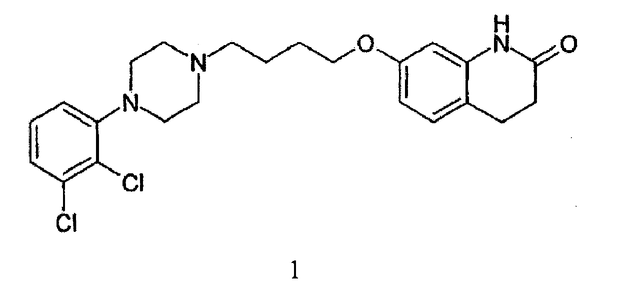

Aripiprazole has shown efficacy in acutely relapsed and longer term schizophrenia and schizoaffective disorder. Aripiprazole is marketed in the United States as Abilify^{™} by Bristol-Myers Squibb Company.

The synthetic route for obtaining aripiprazole and structurally related carbostyril derivatives was first described in U.S. Patent No. 4,734,416 and later in U.S. Patent No. 5,006,528. The synthetic route disclosed in U.S. Patent No. 5,006,528 includes a crystallization step from ethanol. The reported melting point of the resulting crystals was 139-139.5 °C.

U.S. Patent Application Publication No. 2004/0058935 (hereinafter the `935 publication) teaches new crystalline modifications of aripiprazole. These include both a hydrated form, and some anhydrous modifications. The `935 publication teaches that aripiprazole is obtained as a highly hygroscopic product. Since the physical and chemical characteristics of hygroscopic solids can change due to water absorbance, it is desirable to avoid hygroscopic solids in the manufacture of pharmaceutical products.

Table 1 below presents various crystalline forms of aripiprazole, preparation procedures and some characteristic XRPD bands thereof, as taught in the '935 publication.

**Table 1. Characteristic powder diffraction bands and preparation procedures of the aripiprazole forms according to the '935 publication.**

| **aripiprazole form** | **Characteristic powder diffraction bands** | **Preparation procedure** |
|---|---|---|
| Form A | 12.6, 15.4, 17.3, 18.0, 18.6, 22.5, 24.8 | Crystallization from ethanol |
| Form B | 11.0, 16.6, 19.3, 20.3, 22.1 | Heating form A |
| Form C | 12.6, 13.7, 15.4, 18.1, 19.0, 20.6, 23.5, 26.4 | Heating of the anhydrous form to 140 °C |
| Form D | 8.7, 11.6, 16.3, 17.7, 18.6, 20.3, 23.4, 25.0 | Crystallization from toluene |
| Form E | 8.0, 13.7, 14.6, 17.6, 22.5, 24.0 | Crystallization from acetonitrile |
| Form F | 11.3, 13.3, 15.4, 22.8, 25.2, 26.9 | Heating a suspension of aripiprazole in boiling acetone |
| Form G | 10.1, 12.8, 15.2, 17.0, 17.5, 19.1, 20.1, 21.2, 22.4, 23.3, 24.5, 25.8 | Melted glassy aripiprazole left in sealed vessel for weeks or months |

Additional forms of aripiprazole are further described in WO 2004/083183 (hereinafter referred to as the '183 publication). Two crystalline forms of aripiprazole and four crystalline forms of aripiprazole hydrochloride are taught in the ' 183 publication. Table 2 below presents characteristic XRPD bands thereof and preparation procedures of the aripiprazole crystalline forms, as taught in the '183 publication.

The data presented in Tables 1 and 2 suggest that crystalline forms I and II recited in the '183 publication are identical to forms A and D taught in the '935 publication.

**Table 2. Characteristic powder diffraction peaks and preparation procedures of the aripiprazole crystalline forms according to the `183 publication.**

| **aripiprazole form** | **Characteristic X-ray peaks** | **Preparation** |
|---|---|---|
| Form I | 8.7, 11.6, 16.3, 17.7, 18.6, 20.3, 23.4, 24.9 | Crystallization from acetone, ethyl acetate, methanol, and ethanol |
| Form II | 12.7, 15.1, 17.5, 18.2, 18.8, 19.5, 20.6, 21.2, 22.6, 23.3, 24.2, 24.9, 27.6, 30.0, 31.6, 35.8 | Quick evaporation of THF solution, including spray drying |

Other polymorphs of aripiprazole (which are referred to as forms I, III and IV) are disclosed in application WO 2004/106322. Application WO 2005/058835 further discloses aripiprazole forms I, II, VI, VIII, X, XI, XII, XIV, XIX, XX. Application WO 2005/009990 discloses aripiprazole forms III, IV and V, and application US 2005/0277650 discloses a crystalline hydrate of aripiprazole and a process of preparing this hydrate form.

In some cases, different forms of the same drug can exhibit very different solubility, and therefore different dissolution rates (release profile) *in-vivo.*

The above described detailed prior art shows that aripiprazole and its known hydrochloride salt tend to appear in more than one crystalline form, each having different characteristic behavior. These different crystalline forms are known as polymorphs. While polymorphs have the same chemical composition, they can differ in packing and geometrical arrangement, and can exhibit different physical properties such as melting point, shape, color, hardness, bulk density, deformability, stability, dissolution, and the like.

Since each polymorph can have different characteristic behavior, a major problem of using a crystalline polymorphic drug is the difficulty of manufacturing the active pharmaceutical ingredient in a way that consistently and reproducibly produces a solid form having the desired characteristic behavior. An example of the limitations associated with polymorphs is the anti-epilepsy drug carbamazepine, which the US Pharmacopoeia dictates in the monograph the pharmaceutical use of only one specific crystalline form (characterized by its X-ray diffraction pattern). Health authorities in other countries also require assurances for the correct crystalline form of the active ingredient.

In recent years, solid-state properties of drugs have received great focus in the pharmaceutical industry as a major contributing factor to both bioavailability and formulation characteristics. Polymorphism has been considered to be one of the most important solid-state properties that impacts the commercial manufacture of drugs.

Nevertheless, conventional methods for producing crystalline aripiprazole base and aripiprazole hydrochloride suffer from relatively low yields and unstable polymorphism. As such, there is a recognizable need for stable crystalline salts of aripiprazole having pharmaceutical grade purity, and methods for reproducibly obtaining stable salts and in high yield.

The present invention provides such salts and methods, which can be used for producing highly pure aripiprazole or as alternatives to aripiprazole hydrochloride itself.

### BRIEF SUMMARY OF THE INVENTION

The present invention provides new carboxylate salt forms of 7-(4-(4-(2,3-dichlorophenyl)-1-piperazinyl)-butoxy)-3,4-dihydro-2(1*H*)-quinolinone (aripiprazole), which exhibit improved properties. The aripiprazole salts of the present invention can be prepared reproducibly and in high yield by methods known in the art for preparing acid addition salts of active pharmaceutical ingredients, e.g., by treating the active pharmaceutical ingredient with a suitable acid to obtain the desired salt form. Exemplary aripiprazole salts of the present invention include aripiprazole oxalate, aripiprazole benzoate, aripiprazole maleate, aripiprazole malonate, aripiprazole fumarate, aripiprazole L-tartrate, aripiprazole L-malate, and aripiprazole citrate.

In one embodiment, the present invention provides a crystalline solid comprising aripiprazole oxalate characterized by a unique powder X-ray diffraction pattern, e.g., as depicted in Table 3 and Figure 1. The strong diffraction peaks at 11.9, 16.6, 17.4, 18.1, 21.2, 22.8, 24.1, and 25.3 ± 0.2 degrees 2θ are characteristic of this particular form. The aripiprazole oxalate of the present invention also can be characterized by a unique IR spectrum, e.g., as depicted in Figure 2, and by characteristic DSC and TGA curves, e.g., as depicted in Figures 3 and 4, respectively.

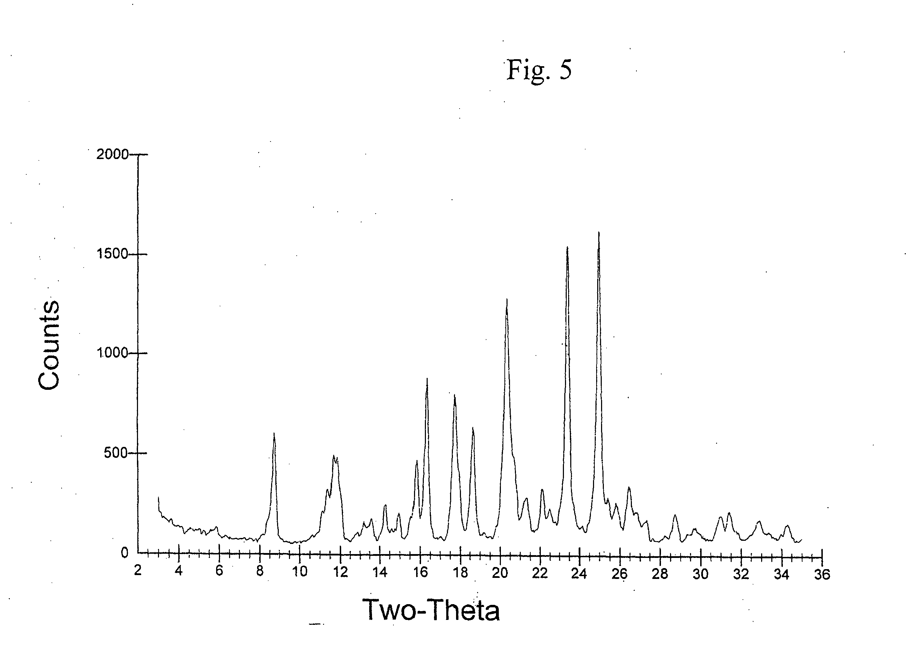

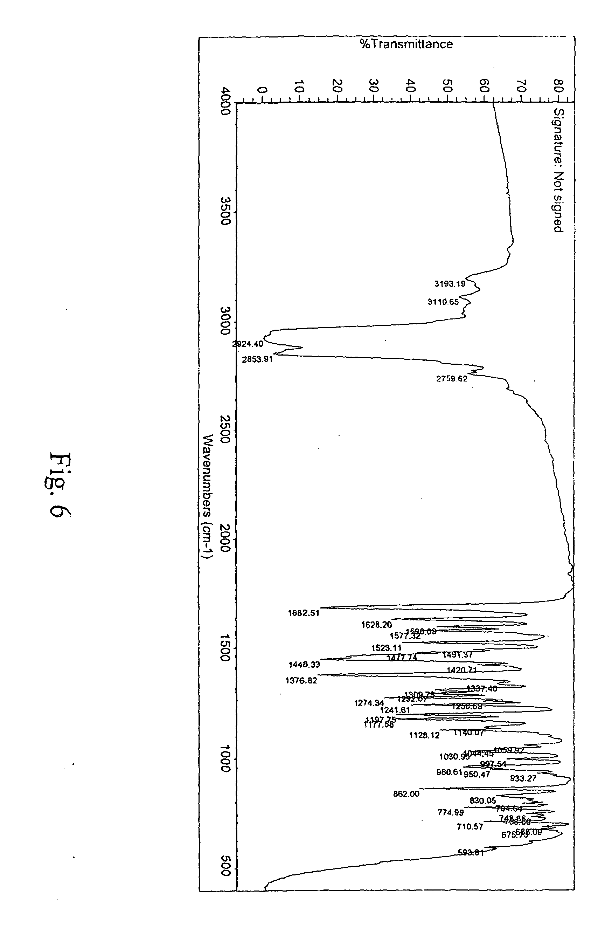

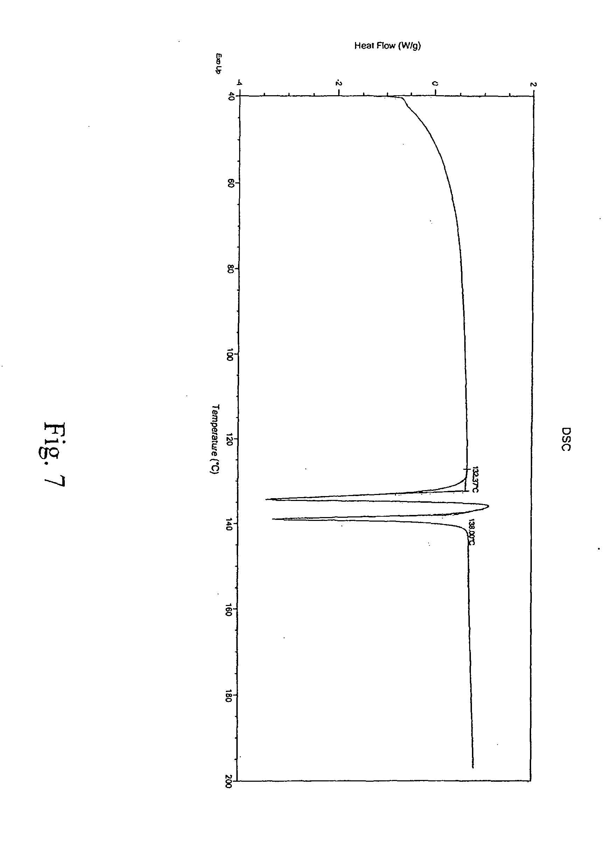

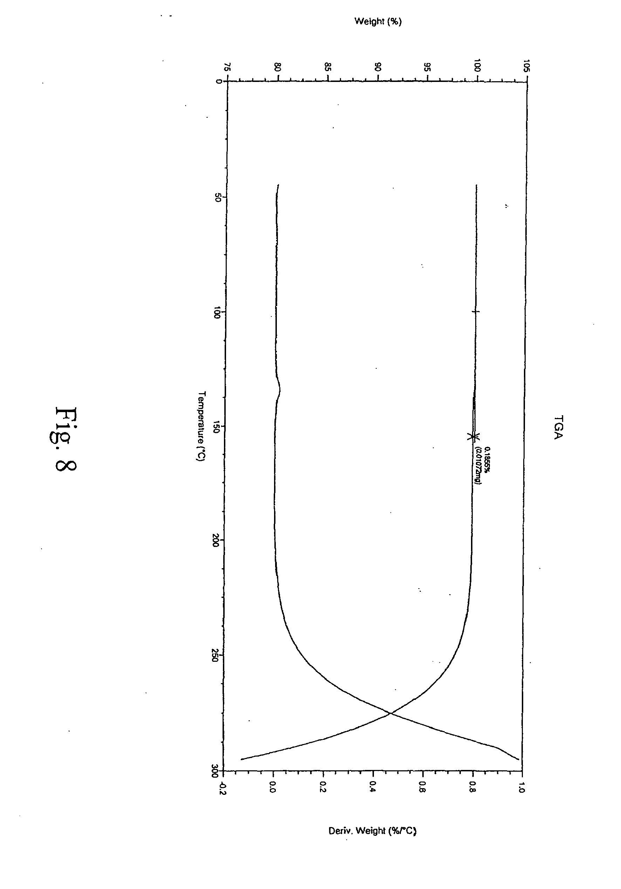

In another embodiment, the present invention provides a crystalline solid comprising aripiprazole benzoate form I, which can be characterized by a unique powder X-ray diffraction pattern, e.g., as depicted in Table 4 and Figure 5. The diffraction peaks at 8.8, 11.7, 15.8, 16.4, 17.8, 18.7, 20.3, 23.4 and 25.0:I: 0.2 degrees 2θ are characteristic of this form. The aripiprazole benzoate form I of the present invention also can be characterized by a unique infra-red spectrum, e.g., as depicted in Figure 6, and by characteristic DSC and TGA curves, e.g., as depicted in Figures 7 and 8, respectively.

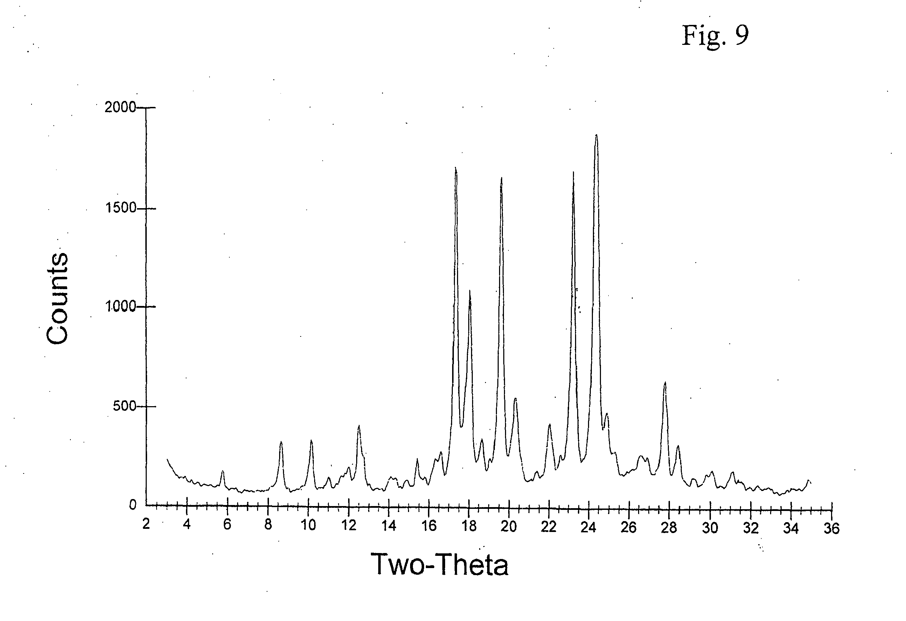

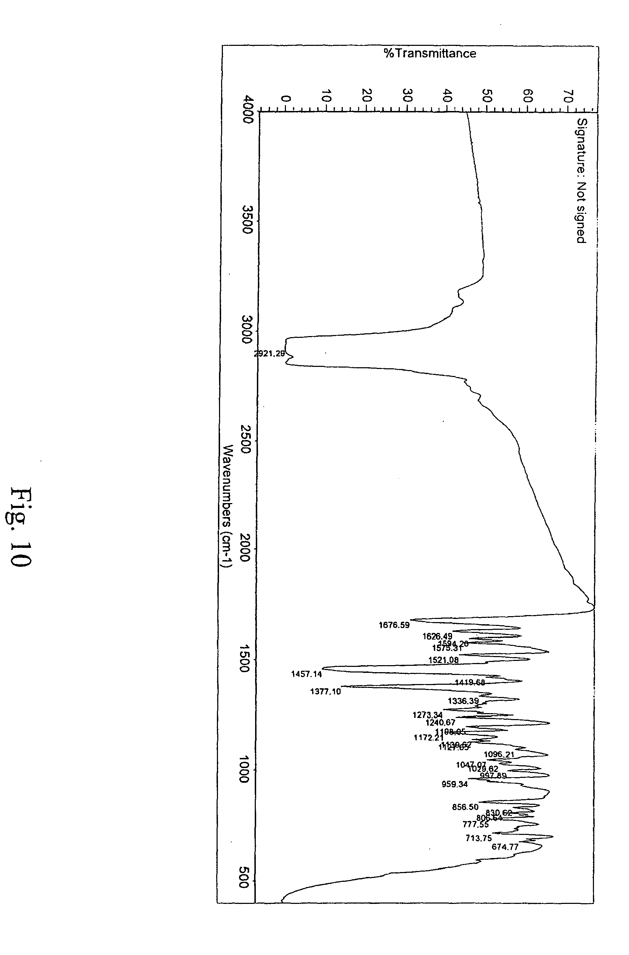

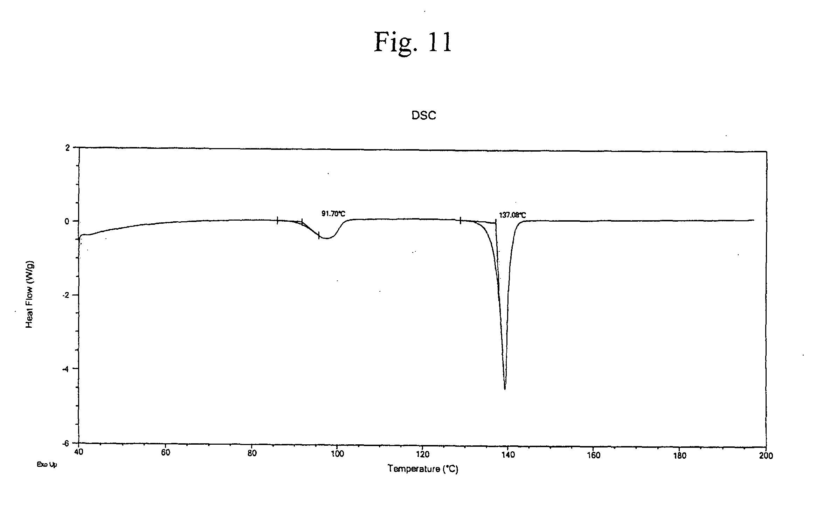

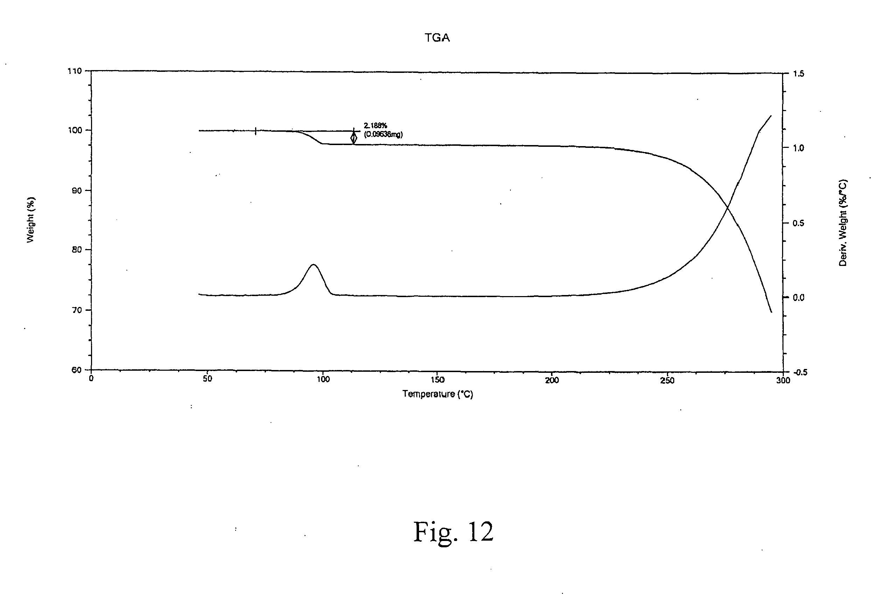

The present invention also provides a crystalline solid comprising aripiprazole benzoate form II, which can be characterized by a unique powder X-ray diffraction pattern, e.g., as depicted in Table 5 and Figure 9. The diffraction peaks at 17.4, 18.1, 19.6, 23.2 and 24.4 ± 0.2 degrees 2θ are characteristic of this form. The aripiprazole benzoate form II of the present invention also can be characterized by a unique infra-red spectrum, e.g., as depicted in Figure 10, and by characteristic DSC and TGA curves, e.g., as depicted in Figures 11 and 12, respectively.

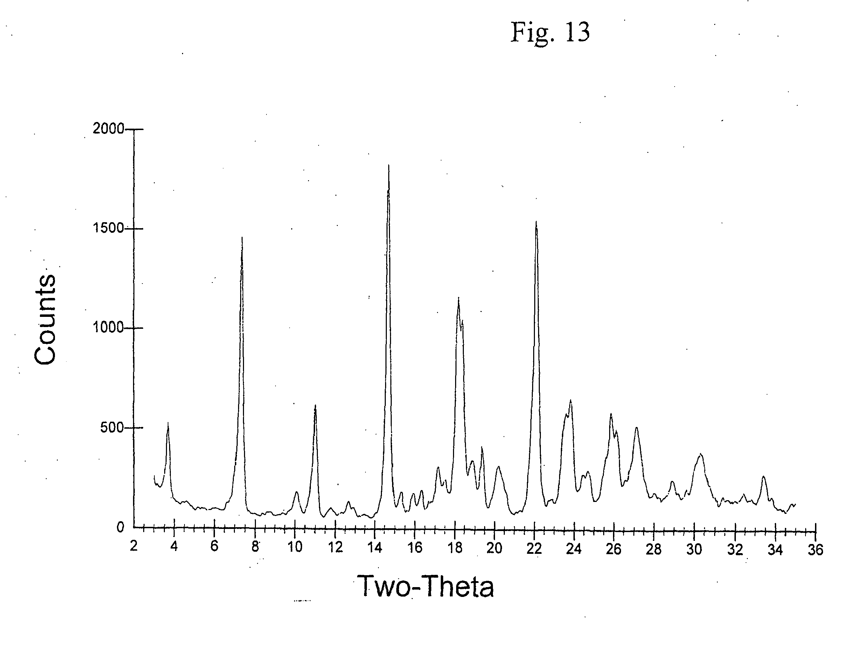

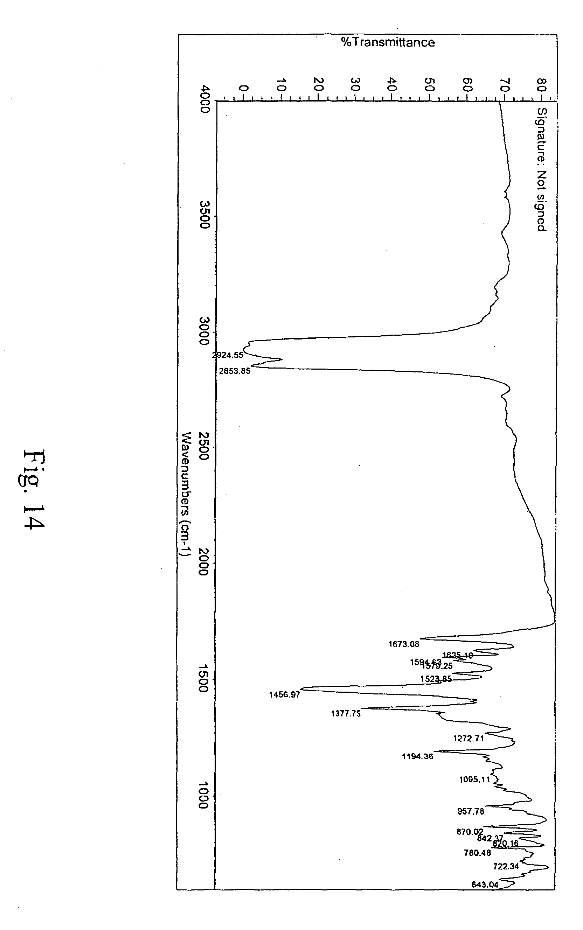

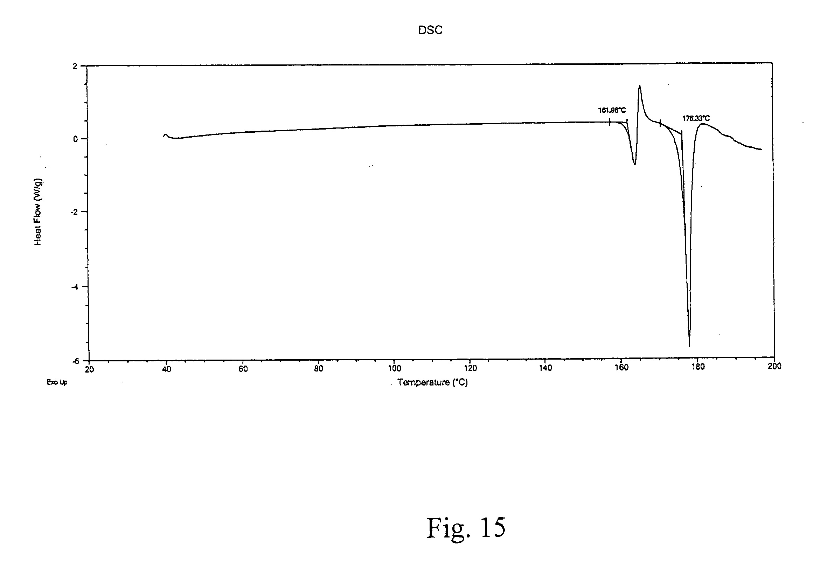

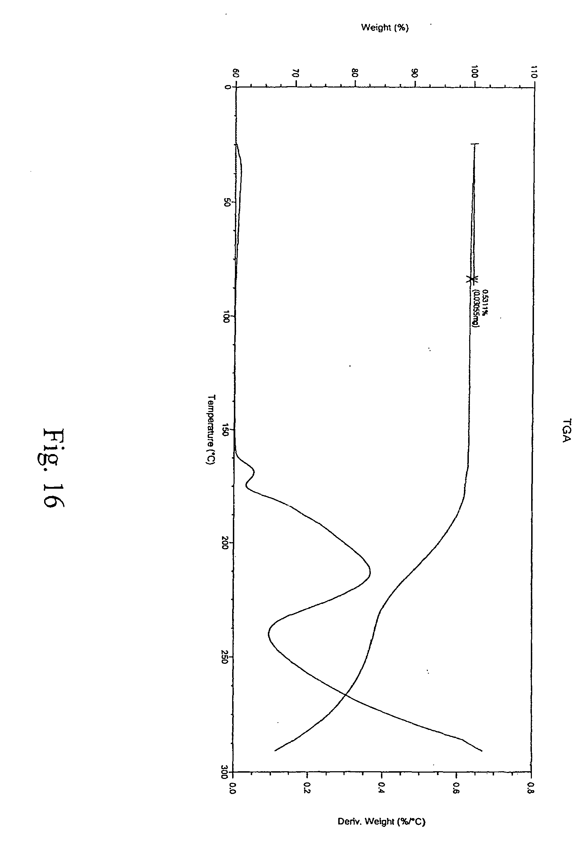

The present invention additionally provides a crystalline solid comprising aripiprazole maleate, which can be characterized by a unique powder X-ray diffraction pattern, e.g., as depicted in Table 6 and Figure 13. The diffraction peaks at 3.7, 7.3, 11.0, 14.7, 18.2, 18.4, 19.4, 22.1, 23.6, 23.8, 25.9, 26.1 and 27.1 ± 0.2 degrees 2θ are characteristic of this particular form. The aripiprazole maleate of the present invention also can be characterized by a unique infra-red spectrum, e.g., as depicted in Figure 14, and by characteristic DSC and TGA curves, e.g., as depicted in Figures 15 and 16, respectively.

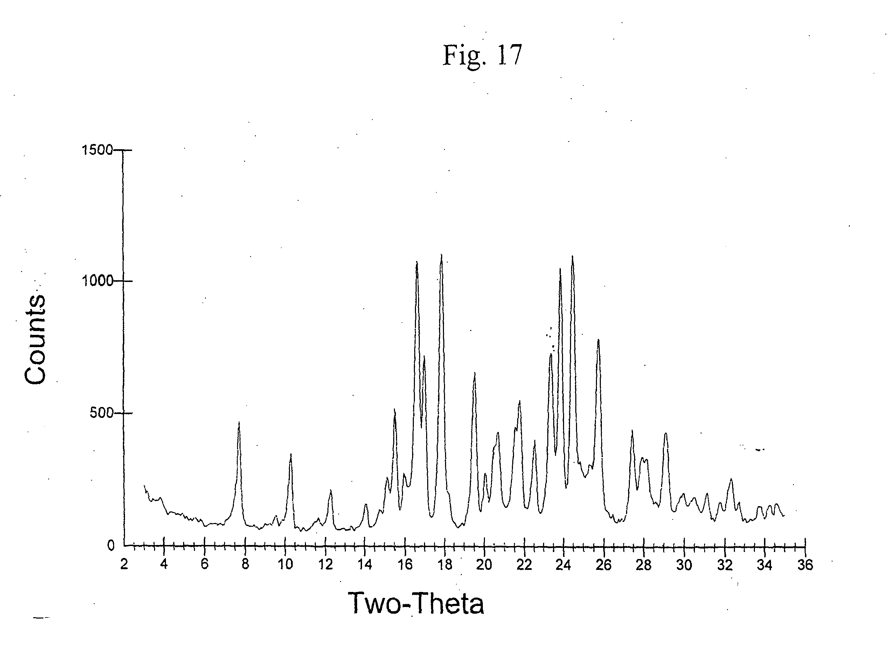

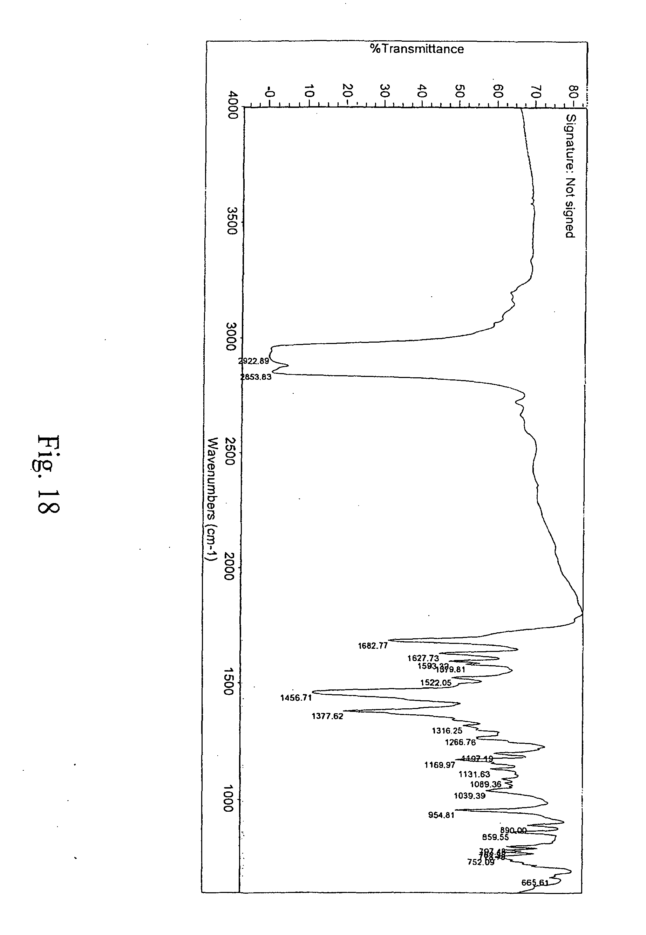

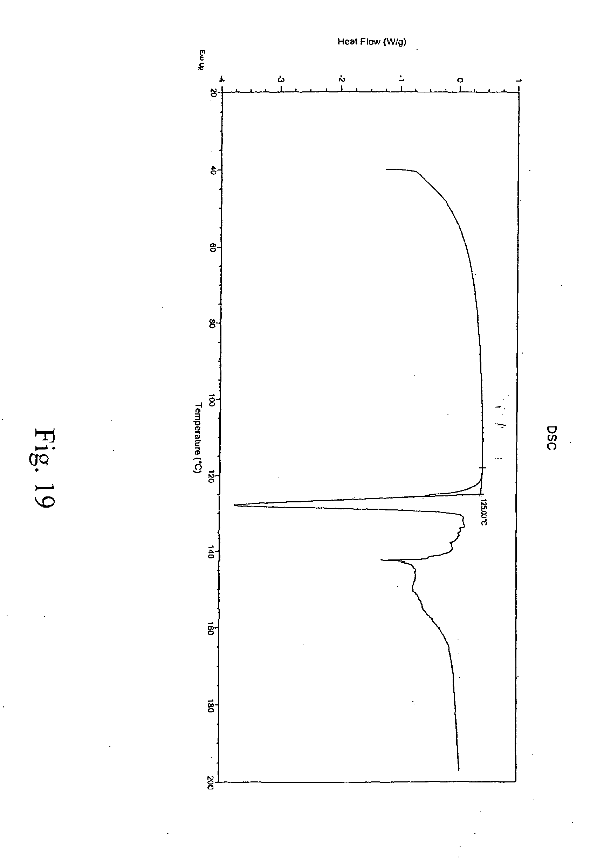

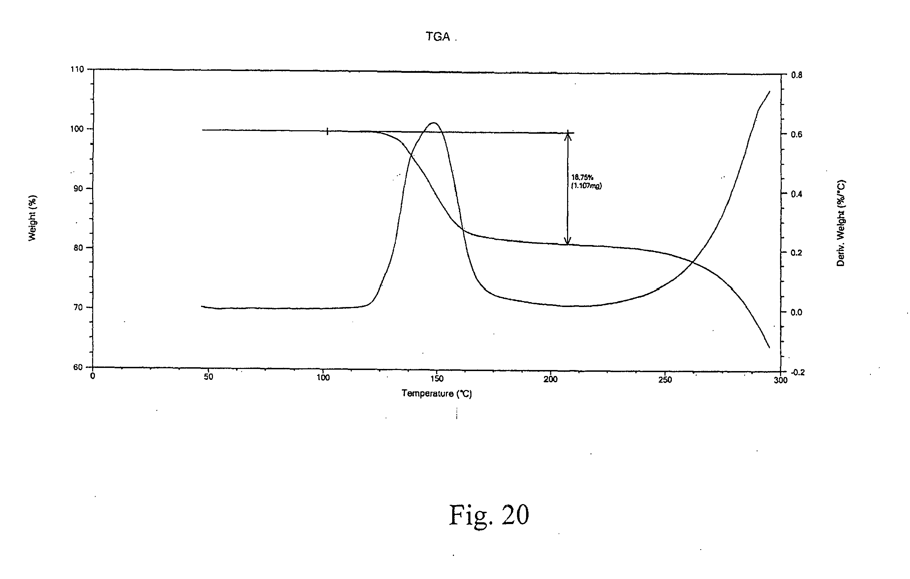

The present invention further provides a crystalline solid comprising aripiprazole malonate, which can be characterized by a unique powder X-ray diffraction pattern, e.g., as depicted in Table 7 and Figure 17. The diffraction peaks at 7.7, 10.3, 15.5, 16.7, 17.0, 17.9, 19.5, 20.7, 21.5, 21.8, 22.5, 23.4, 23.9, 24.5, 25.8, 27.4 and 29.1 ± 0.2 degrees 2θ are characteristic of this particular form. The aripiprazole malonate of the present invention also can be characterized by a unique infra-red spectrum, e.g., as depicted in Figure 18, and by characteristic DSC and TGA curves, e.g., as depicted in Figures 19 and 20, respectively.

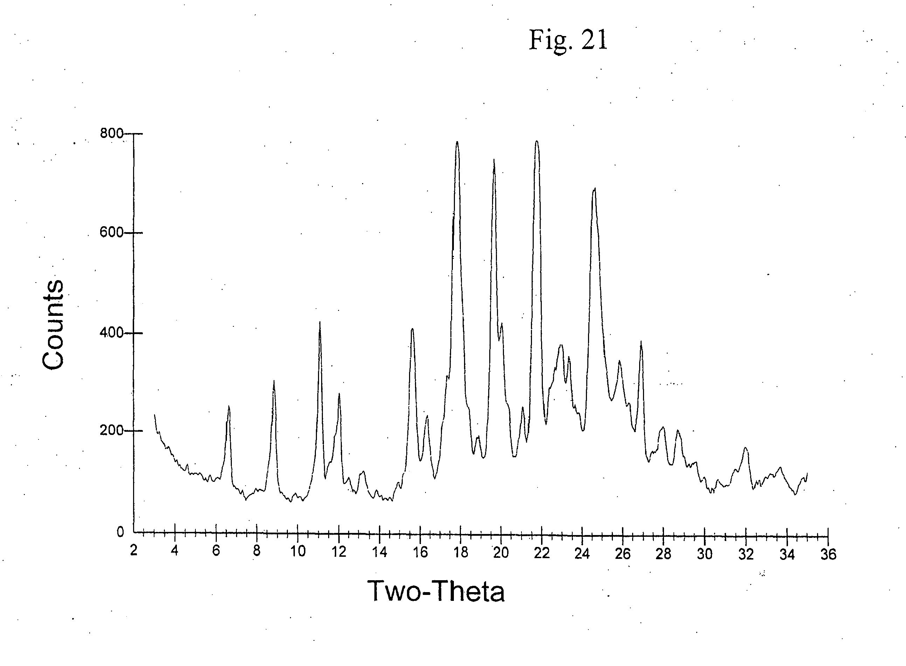

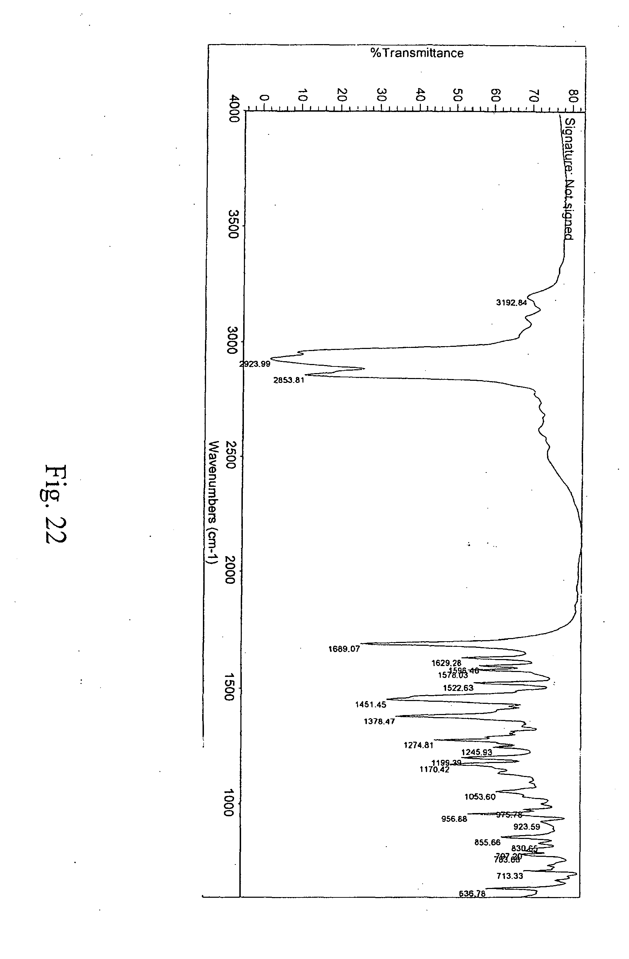

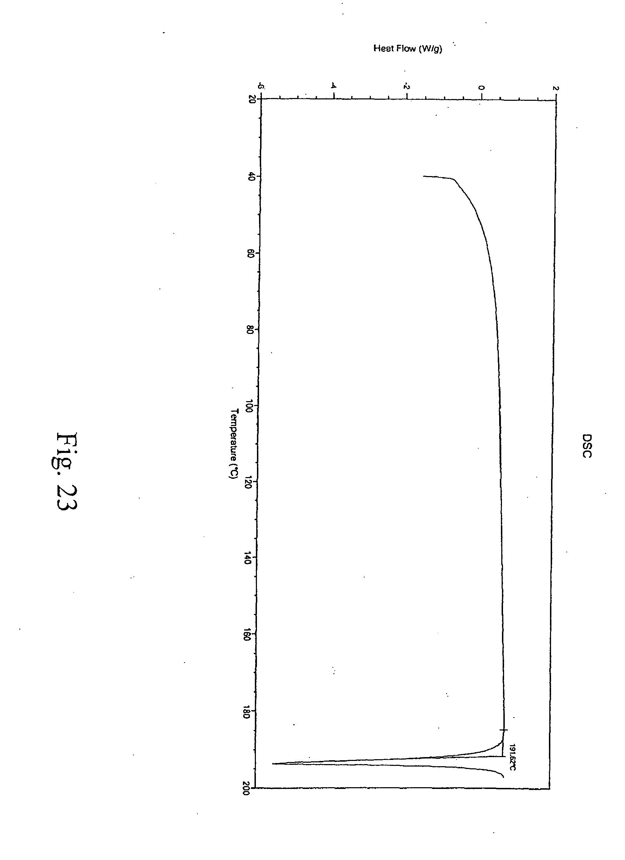

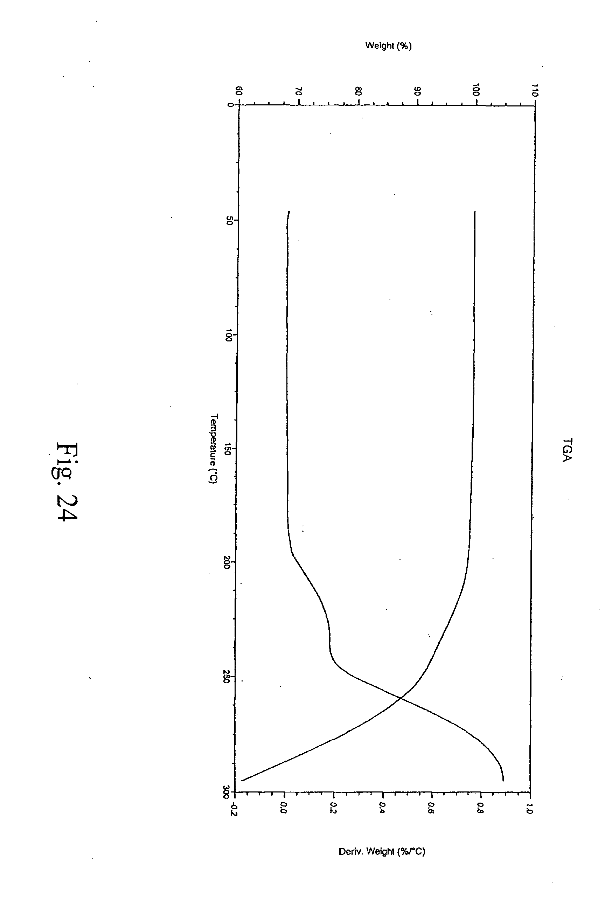

The present invention still further provides a crystalline solid comprising aripiprazole fumarate, which can be characterized by a unique powder X-ray diffraction pattern, e.g., as depicted in Table 8 and Figure 21. The diffraction peaks at 6.6, 8.8, 11.1, 12.0, 15.6, 17.4, 17.8, 19.7, 20.1, 21.8, 23.0, 23.4, 24.6, 25.8 and 26.9 ± 0.2 degrees 2θ are characteristic of this particular form. The aripiprazole fumarate of the present invention also can be characterized by a unique infra-red spectrum, e.g., as depicted in Figure 22, and by characteristic DSC and TGA curves, e.g., as depicted in Figures 23 and 24, respectively.

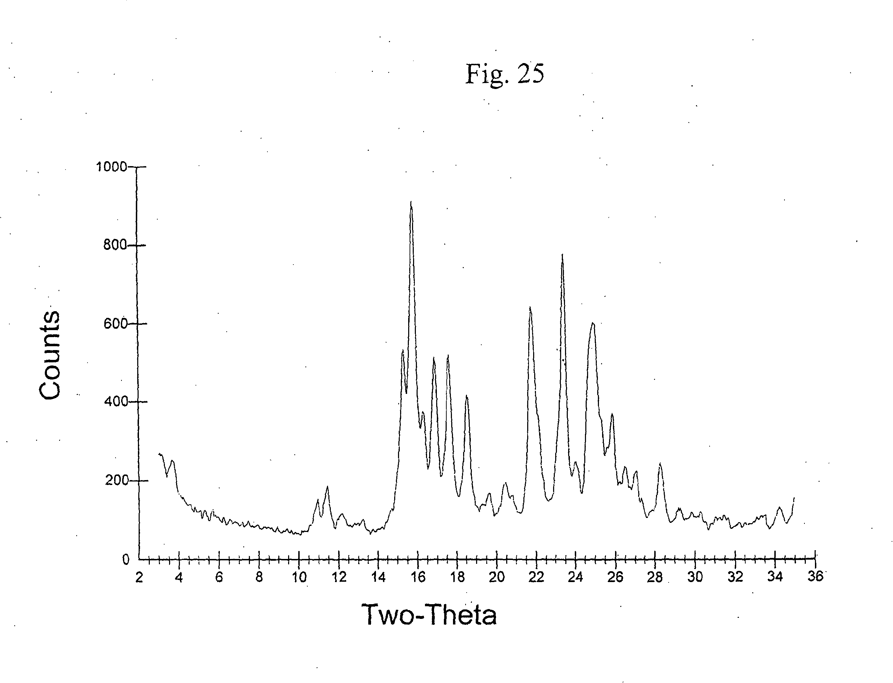

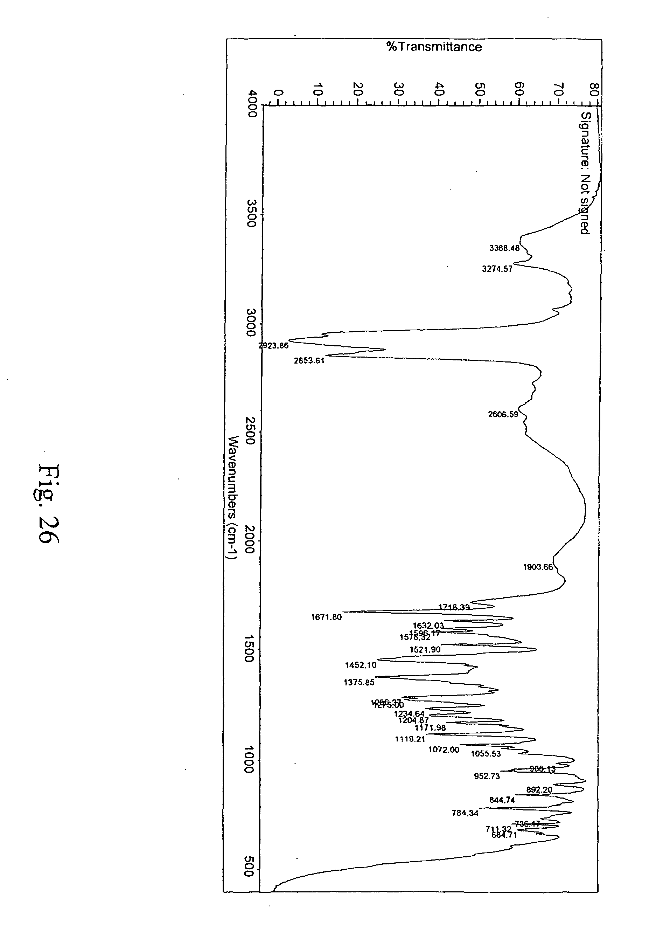

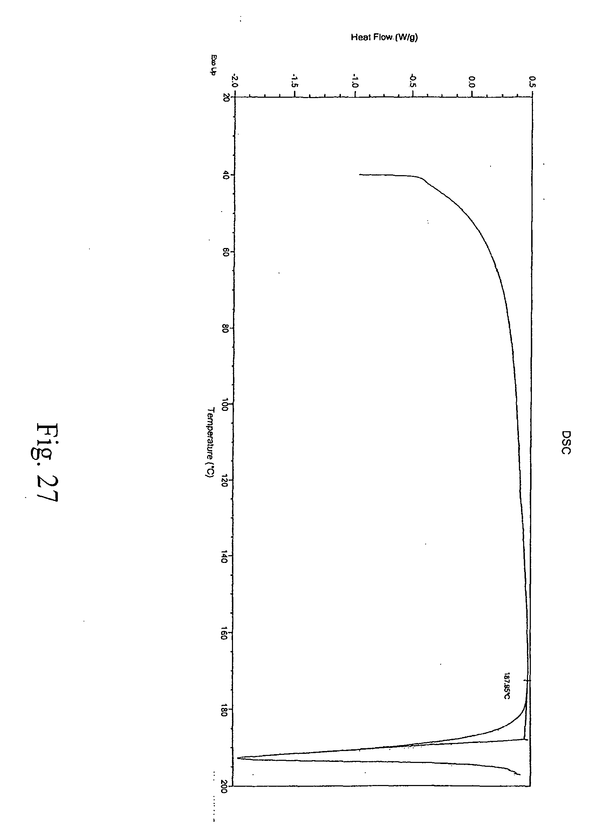

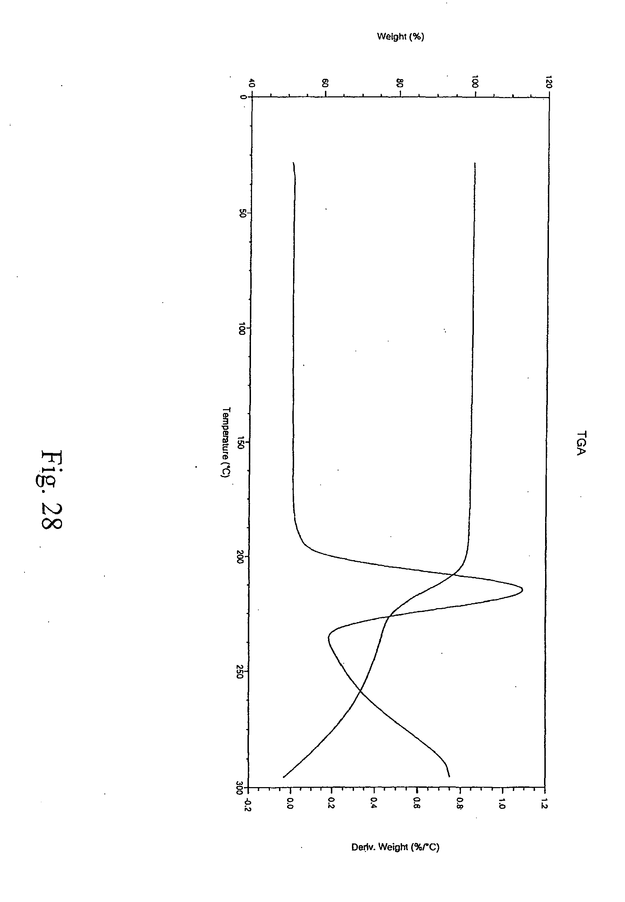

The present invention moreover provides a crystalline solid comprising aripiprazole L-tartrate, which is characterized by a unique powder X-ray diffraction pattern, e.g., as depicted in Table 9 and Figure 25. The diffraction peaks at 15.3, 15.8, 16.3, 16.9, 17.6, 18.6, 21.8, 23.4, 25.0 and 25.9 ± 0.2 degrees 2θ are characteristic of this form. The aripiprazole L-tartrate of the present invention also can be characterized by a unique infra-red spectrum, e.g., as depicted in Figure 26, and by characteristic DSC and TGA curves, e.g., as depicted in Figures 27 and 28, respectively.

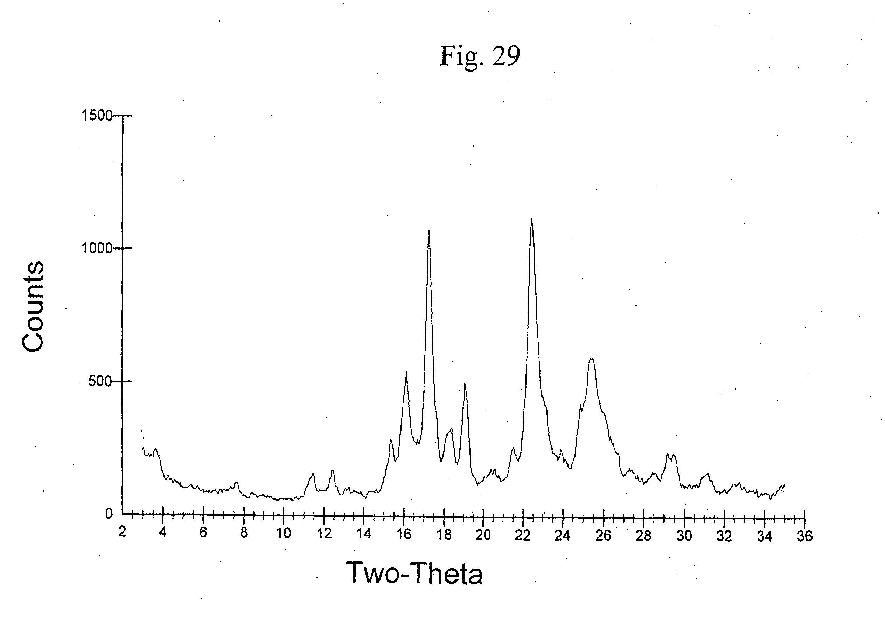

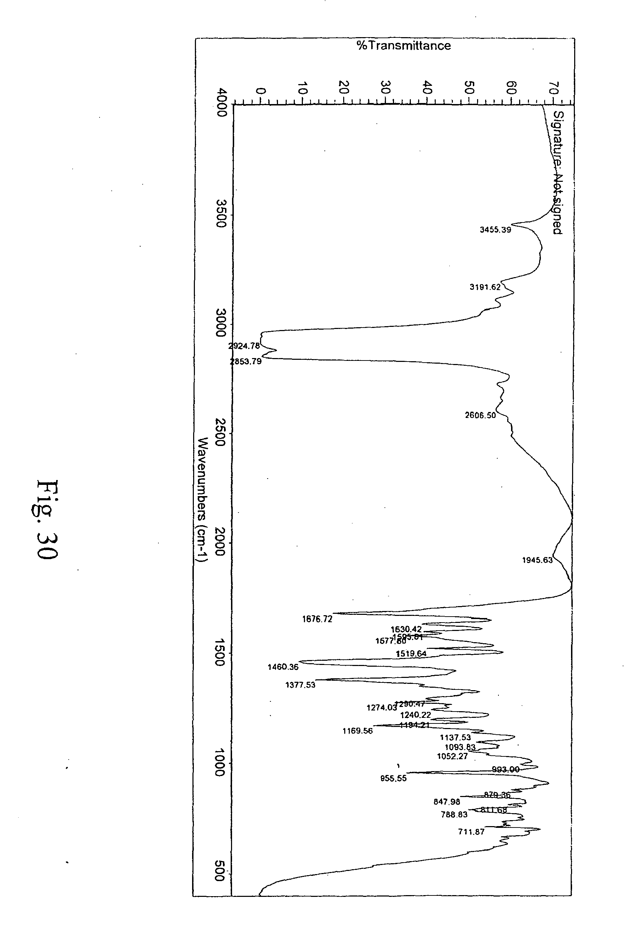

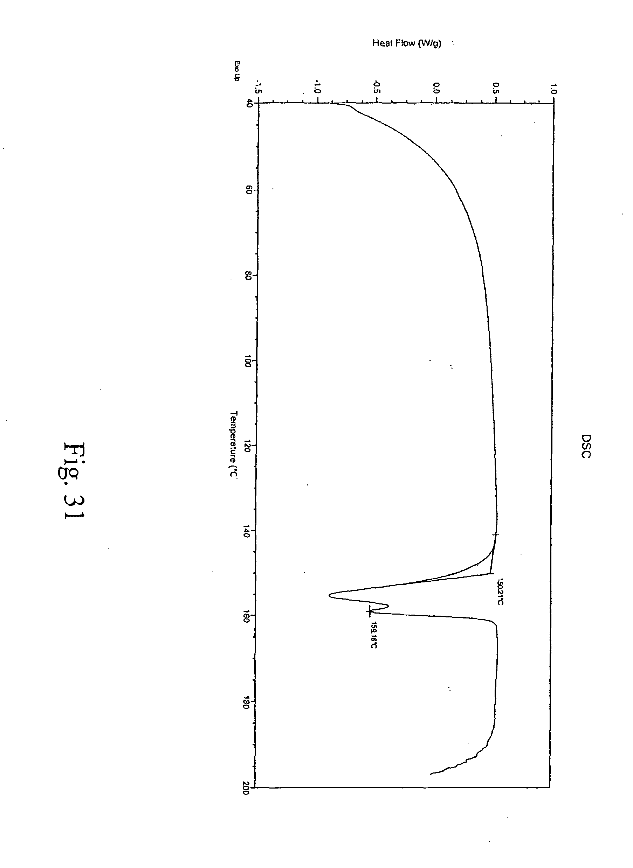

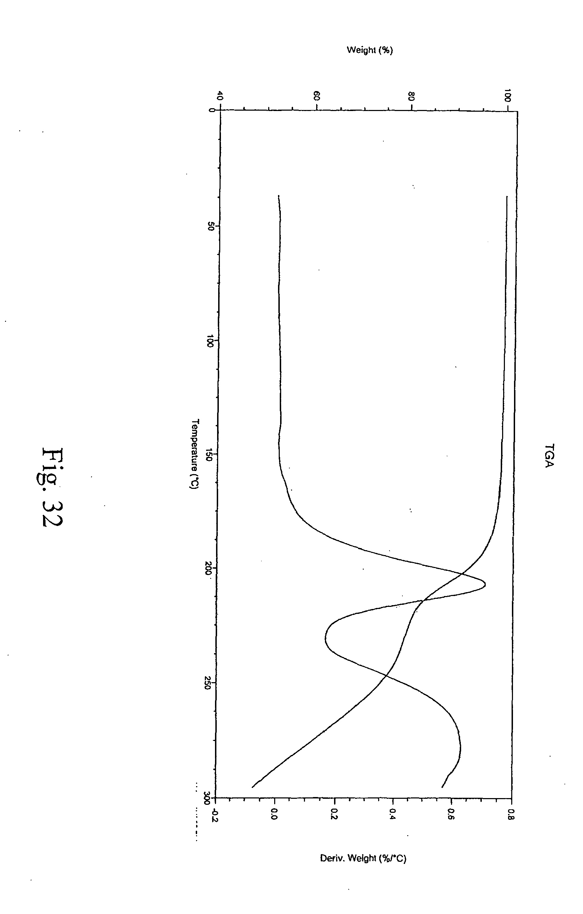

In another embodiment, the present invention provides a crystalline solid comprising aripiprazole L-malate, which can be characterized by a unique powder X-ray diffraction pattern, e.g., as depicted in Table 10 and Figure 29. The diffraction peaks at 15.4, 16.2, 17.3, 18.4, 19.0, 22.4, and 25.3 ± 0.2 degrees 2θ are characteristic of this particular form. The aripiprazole L-malate also can be characterized by a unique infra-red spectrum, e.g., as depicted in Figure 30, and by characteristic DSC and TGA curves, e.g., as depicted in Figures 31 and 32, respectively.

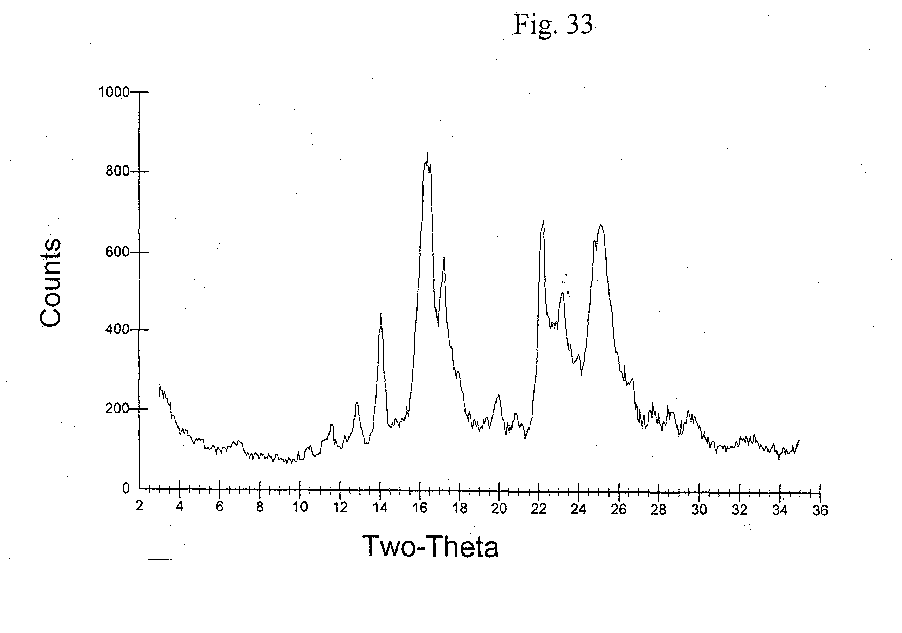

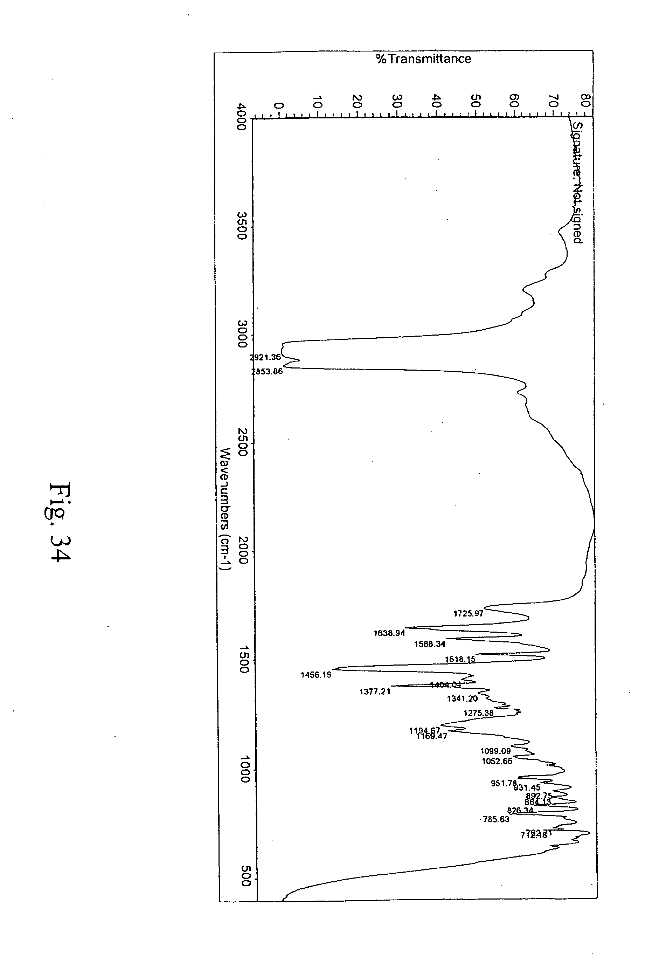

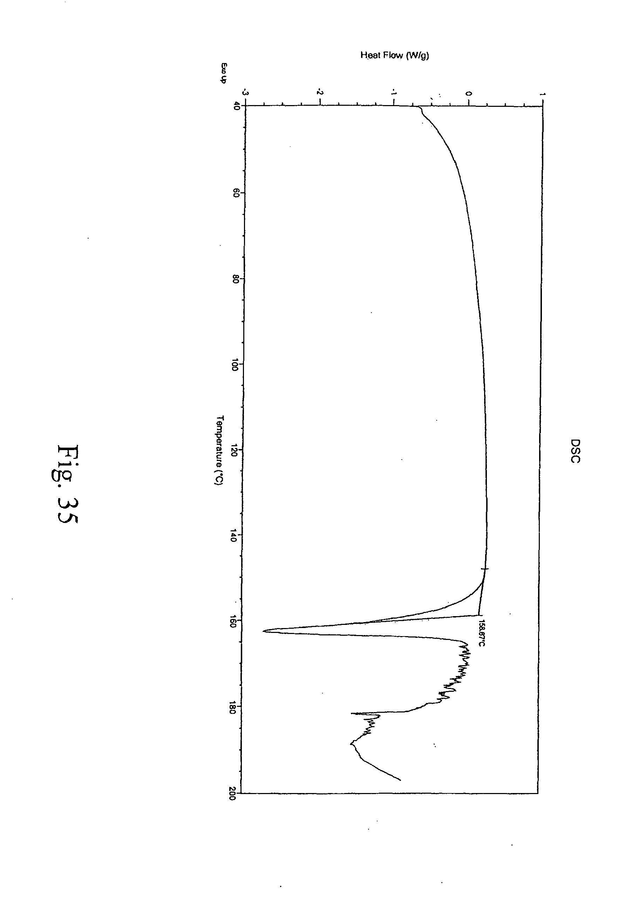

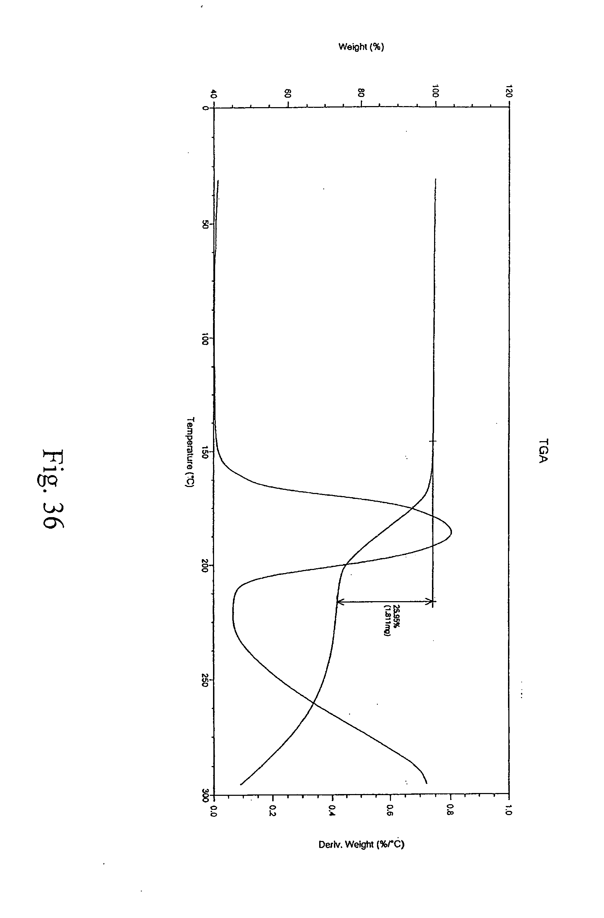

In yet another embodiment, the present invention provides a crystalline solid comprising aripiprazole citrate, which can be characterized by a unique powder X-ray diffraction pattern, e.g., as depicted in Table 11 and Figure 33. The diffraction peaks at 14.0, 16.4, 17.2, 17.9, 22.2, 23.2, 25.1 and 26.7 ± 0.2 degrees 2θ are characteristic of this particular form. The aripiprazole citrate also can be characterized by a unique infra-red spectrum, e.g., as depicted in Figure 34, and by DSC and TGA curves, e.g., as depicted in Figures 35 and 36, respectively.

The aripiprazole salts of the present invention can be prepared, e.g., by suspending aripiprazole, obtained by any suitable method known in the art, in an organic solvent, e.g., a C₁-C₄ alcohol (e.g., ethanol) or a water-immiscible solvent (e.g., ethyl acetate), optionally with heating, preferably to reflux temperature, and adding a solution of an organic acid in, e.g., a C₁-C₄ alcohol (e.g., ethanol), subsequently crystallizing the salt, e.g., by cooling, and isolating the product by any suitable method, e.g., by filtration.

The present invention further provides a process for preparing aripiprazole salts. The process of the present invention preferably comprises:
suspending aripiprazole in an organic solvent;
heating the mixture to elevated temperature, preferably to reflux, to form a solution;
adding a solution of an organic or a mineral acid in, e.g., a Cₗ-C₄ alcohol or a water-immiscible solvent, e.g., ethyl acetate;
allowing the mixture to cool sufficiently to produce crystals of an aripiprazole salt;
collecting the obtained crystals by filtration; and
optionally washing the crystals and drying, optionally under reduced pressure.

The present invention further provides a method for preparing highly pure aripiprazole base in high yield using the crystalline aripiprazole salts of the present invention. In one embodiment, the process of the present invention for producing highly pure aripiprazole comprises:
providing a two-phase mixture comprising the aripiprazole salt, an organic solvent and water;
adding a base, preferably with stirring and heating to an elevated temperature;
separating the layers, adding water to the organic phase, preferably with stirring and heating;
distilling to remove at least a portion of the solvents; and
adding a second organic solvent and cooling to precipitate aripiprazole, which can be islolated, e.g., by filtration.

### BRIEF DESCRIPTION OF THE DRAWINGS

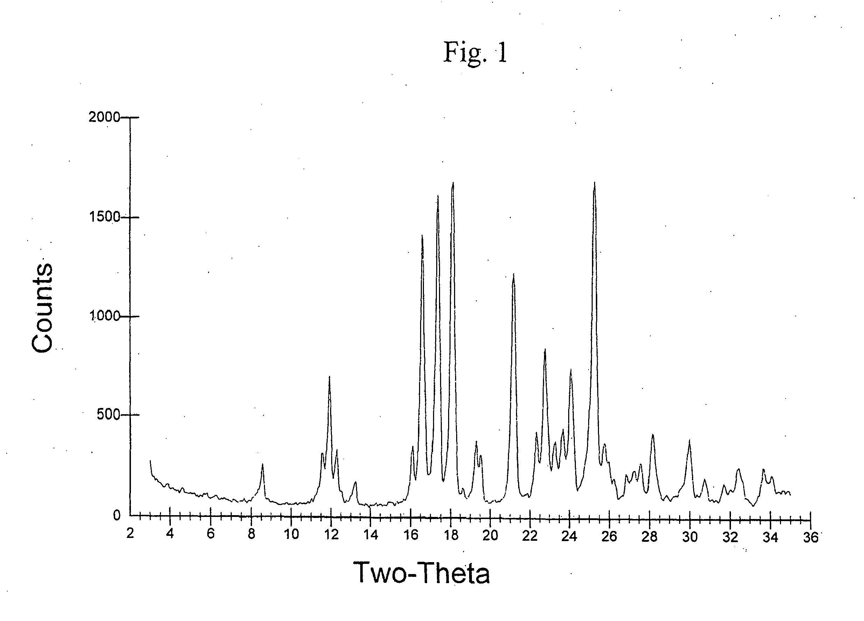

Figure 1 depicts the powder X-ray diffraction pattern of aripiprazole oxalate.

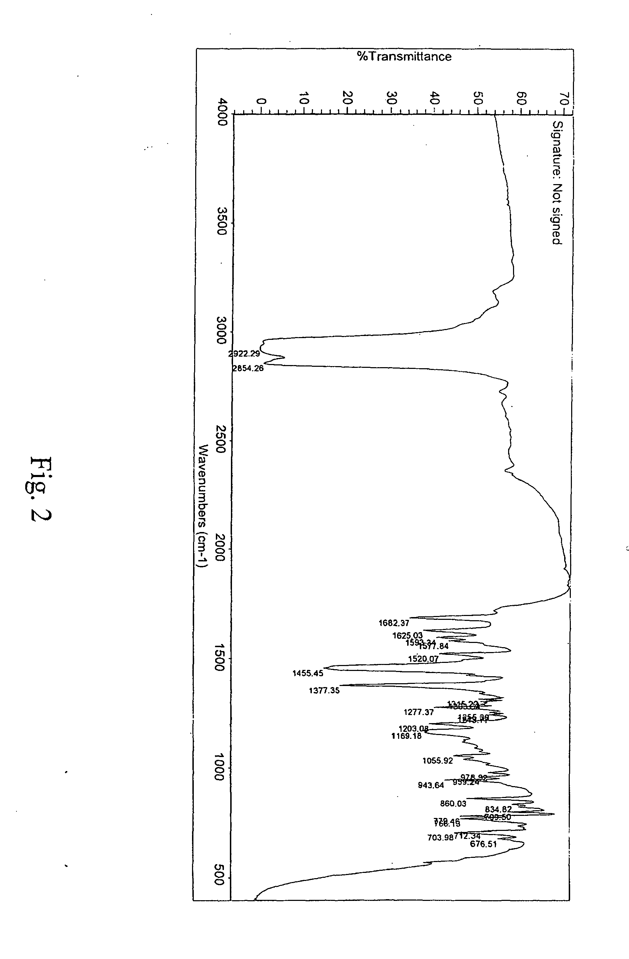

Figure 2 depicts the infra-red spectrum of aripiprazole oxalate.

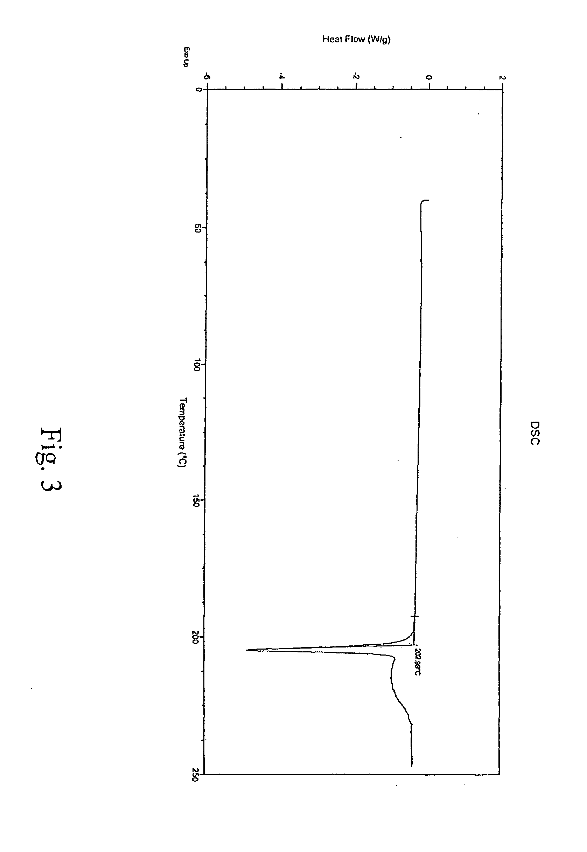

Figure 3 depicts the differential scanning calorimetry (DSC) curve of aripiprazole oxalate.

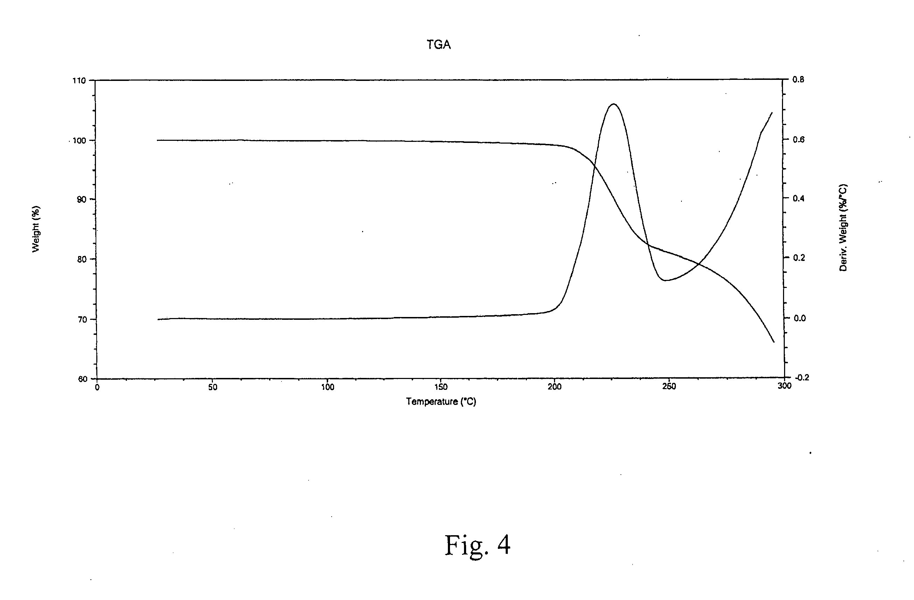

Figure 4 depicts the thermogravimetric analysis (TGA) curve of aripiprazole oxalate.

Figure 5 depicts the powder X-ray diffraction pattern of aripiprazole benzoate form I.

Figure 6 depicts the infra-red spectrum of aripiprazole benzoate form I.

Figure 7 depicts the DSC curve of aripiprazole benzoate form I.

Figure 8 depicts the thermogravimetric analysis (TGA) curve of aripiprazole benzoate form I.

Figure 9 depicts the powder X-ray diffraction pattern of aripiprazole benzoate form II.

Figure 10 depicts the infra-red spectrum of aripiprazole benzoate form II.

Figure 11 depicts the differential scanning calorimetry (DSC) curve aripiprazole benzoate form II.

Figure 12 depicts the thermogravimetric analysis (TGA) curve of aripiprazole benzoate form II.

Figure 13 depicts the powder X-ray diffraction pattern of aripiprazole maleate.

Figure 14 depicts the infra-red spectrum of aripiprazole maleate.

Figure 15 depicts the differential scanning calorimetry (DSC) curve aripiprazole maleate.

Figure 16 depicts the thermogravimetric analysis (TGA) curve of aripiprazole maleate.

Figure 17 depicts the X-ray diffraction pattern of aripiprazole malonate.

Figure 18 depicts the infra-red spectrum of aripiprazole malonate.

Figure 19 depicts the differential scanning calorimetry (DSC) curve of aripiprazole malonate.

Figure 20 depicts the thermogravimetric analysis (TGA) curve of aripiprazole malonate.

Figure 21 depicts the powder X-ray diffraction pattern of aripiprazole fumarate.

Figure 22 depicts the infra-red spectrum of aripiprazole fumarate.

Figure 23 depicts the differential scanning calorimetry (DSC) curve of aripiprazole fumarate.

Figure 24 depicts the thermogravimetric analysis (TGA) curve of aripiprazole fumarate.

Figure 25 depicts the powder X-ray diffraction pattern of aripiprazole L-tartrate.

Figure 26 depicts the infra-red spectrum of aripiprazole L-tartrate.

Figure 27 depicts the differential scanning calorimetry (DSC) curve of aripiprazole L-tartrate.

Figure 28 depicts the thermogravimetric analysis (TGA) curve of aripiprazole L-tartrate.

Figure 29 depicts the powder X-ray diffraction pattern of aripiprazole L-malate.

Figure 30 depicts the infra-red spectrum of aripiprazole L-malate.

Figure 31 depicts the differential scanning calorimetry (DSC) curve of aripiprazole L-malate.

Figure 32 depicts the thermogravimetric analysis (TGA) curve of aripiprazole L-malate.

Figure 33 depicts the powder X-ray diffraction pattern of aripiprazole citrate.

Figure 34 depicts the infra-red spectrum of aripiprazole citrate.

Figure 35 depicts the differential scanning calorimetry (DSC) curve of aripiprazole citrate.

Figure 36 depicts the thermogravimetric analysis (TGA) curve of aripiprazole citrate.

### DETAILED DESCRIPTION OF THE INVENTION

The present invention provides novel salt forms of 7-(4-(4-(2,3-dichlorophenyl)-1-piperazinyl)-butoxy)-3,4-dihydro-2(1*H*)-quinolinone (aripiprazole), which exhibit improved properties over conventional salt forms. The aripiprazole salts of the present invention can be prepared by methods known in the art for preparing acid addition salts of active pharmaceutical ingredients, e.g., by treating the active pharmaceutical ingredient (e.g., in the form of its free base) with a suitable acid to obtain the salt form. Exemplary aripiprazole salts of the present invention include crystalline forms of one or more of the following: aripiprazole oxalate, aripiprazole benzoate, aripiprazole maleate, aripiprazole malonate, aripiprazole fumarate, aripiprazole L-tartrate, aripiprazole L-malate, or aripiprazole citrate.

In one embodiment, the present invention provides a crystalline solid comprising aripiprazole oxalate characterized by a unique powder X-ray diffraction pattern, e.g., as depicted in Table 3 and Figure 1.

**Table 3 - Aripiprazole oxalate - Powder X-ray diffraction peak positions and intensities.**

| 2θ degrees | I/I₀ | | 2θ degrees | I/I₀ |
|---|---|---|---|---|
| 8.6 | 11.3 | | 23.7 | 17.8 |
| 11.6 | 13.1 | | 24.1 | 30.9 |
| 11.9 | 32.3 | | 25.3 | 100.0 |
| 12.3 | 14.3 | | 25.8 | 13.3 |
| 13.2 | 6.2 | | 26.2 | 4.8 |
| 16.1 | 14.2 | | 26.9 | 6.7 |
| 16.6 | 68.8 | | 27.3 | 7.3 |
| 17.4 | 85.0 | | 27.6 | 9.0 |
| 18.1 | 91.7 | | 28.2 | 16.0 |
| 19.3 | 15.3 | | 30.0 | 15.6 |
| 19.6 | 11.0 | | 30.7 | 6.0 |
| 21.2 | 53.1 | | 31.7 | 4.8 |
| 22.3 | 17.0 | | 32.4 | 8.2 |
| 22.8 | 37.8 | | 33.7 | 8.6 |
| 23.3 | 14.8 | | 34.1 | 6.8 |

The strong diffraction peaks at 11.9, 16.6, 17.4, 18.1, 21.2, 22.8, 24.1, and 25.3 ± 0.2 degrees 2θ are characteristic of this particular form.

The aripiprazole oxalate of the present invention also can be characterized by a unique IR spectrum, e.g., as depicted in Figure 2. The band at 1170 cm⁻¹, and the doublet at 768 cm⁻¹ and 779 cm⁻¹ are characteristic of this particular form. The aripiprazole oxalate of the present invention also can be characterized by characteristic DSC and TGA curves, e.g., as depicted in Figures 3 and 4, respectively.

In another embodiment, the present invention provides a crystalline solid comprising aripiprazole benzoate form I, which can be characterized by a unique powder X-ray diffraction pattern, e.g., as depicted in Table 4 and Figure 5.

**Table 4 - Aaripiprazole benzoate form I - Powder X-ray diffraction peak positions and intensities.**

| 2θ degrees | I/I₀ | | 2θ degrees | I/I₀ |
|---|---|---|---|---|
| 5.9 | 2.7 | | 21.3 | 13.1 |
| 8.8 | 35.2 | | 22.1 | 15.9 |
| 11.1 | 9.6 | | 22.5 | 9.6 |
| 11.4 | 16.9 | | 23.4 | 98.2 |
| 11.7 | 26.9 | | 25.0 | 100.0 |
| 12.9 | 3.6 | | 25.8 | 12.2 |
| 13.2 | 6.8 | | 26.5 | 17.0 |
| 13.6 | 7.4 | | 26.9 | 9.2 |
| 14.3 | 12.1 | | 27.3 | 7.0 |
| 15.0 | 8.9 | | 28.7 | 8.9 |
| 15.8 | 25.2 | | 29.7 | 4.4 |
| 16.4 | 52.1 | | 31.0 | 7.6 |
| 17.8 | 45.8 | | 31.4 | 9.1 |
| 18.7 | 35.4 | | 32.9 | 6.5 |
| 20.3 | 75.9 | | 34.3 | 5.0 |

The diffraction peaks at 8.8, 11.7, 15.8, 16.4, 17.8, 18.7, 20.3, 23.4 and 25.0 ± 0.2 degrees 2θ are characteristic of this form.

The aripiprazole benzoate form I of the present invention also can be characterized by a unique infra-red spectrum, e.g., as depicted in Figure 6. The bands at 1682 cm⁻¹, 1310 cm⁻¹, 1293 cm⁻¹, 1274 cm⁻¹, and 862 cm⁻¹ are characteristic of this form.

The aripiprazole benzoate form I of the present invention also can be characterized by a characteristic DSC curve, e.g., as depicted in Figure 7, where the transition at 132°C is characteristic; however, melting is not observed at this temperature. The aripiprazole benzoate form I of the present invention also can be
characterized by characteristic TGA curve, e.g., as depicted in Figure 8. The present invention further provides a process for preparing aripiprazole benzoate form I by crystallization from ethyl acetate.

The present invention additionally provides a crystalline solid comprising aripiprazole benzoate form II, which can be characterized by a unique powder X-ray diffraction pattern, e.g., as depicted in Table 5 and Figure 9.

**Table 5 - Aripiprazole benzoate form II - Powder X-ray diffraction peak positions and intensities.**

| 2θ degrees | I/I₀ | | 2θ degrees | I/I₀ |
|---|---|---|---|---|
| 5.8 | 3.6 | | 19.6 | 57.9 |
| 8.7 | 8.8 | | 20.3 | 13.8 |
| 10.2 | 9.8 | | 22.0 | 9.5 |
| 11.0 | 2.7 | | 22.6 | 4.5 |
| 12.0 | 4.1 | | 23.2 | 60.2 |
| 12.5 | 11.1 | | 24.4 | 100.0 |
| 12.7 | 5.7 | | 24.9 | 11.5 |
| 14.1 | 2.2 | | 26.6 | 4.7 |
| 14.9 | 1.2 | | 26.9 | 4.3 |
| 15.4 | 5.4 | | 27.8 | 18.4 |
| 16.3 | 4.3 | | 28.4 | 7.5 |
| 16.6 | 5.5 | | 29.1 | 2.0 |
| 17.4 | 92.3 | | 29.9 | 2.6 |
| 18.1 | 33.7 | | 30.1 | 3.5 |
| 18.7 | 7.3 | | 31.1 | 3.7 |

The diffraction peaks at 17.4, 18.1, 19.6, 23.2 and 24.4 ± 0.2 degrees 2θ are characteristic of this form.

The aripiprazole benzoate form II of the present invention also can be characterized by a unique infra-red spectrum, e.g., as depicted in Figure 10, wherein the band at 1676 cm⁻¹ is characteristic of this form. The aripiprazole benzoate form II of the present invention also can be characterized by characteristic DSC and TGA curves, e.g., as depicted in Figures 11 and 12, respectively, showing that this form is an ethanol solvate, containing about 2% of ethanol. The present invention also provides a process for preparing aripiprazole benzoate form II by crystallization from ethanol.

The present invention additionally provides a crystalline solid comprising aripiprazole maleate, which can be characterized by a unique powder X-ray diffraction pattern, e.g., as depicted in Table 6 and Figure 13.

**Table 6 - Aripiprazole maleate - Powder X-ray diffraction peak positions and intensities.**

| 2θ degrees | I/I₀ | | 2θ degrees | I/I₀ |
|---|---|---|---|---|
| 3.7 | 21.3 | | 20.2 | 13.7 |
| 7.3 | 80.2 | | 22.1 | 81.4 |
| 10.0 | 6.8 | | 22.9 | 3.7 |
| 11.0 | 34.3 | | 23.6 | 26.0 |
| 11.8 | 2.2 | | 23.8 | 29.6 |
| 12.7 | 4.3 | | 24.7 | 9.7 |
| 14.7 | 100.0 | | 25.9 | 25.3 |
| 15.3 | 7.7 | | 26.1 | 21.1 |
| 15.9 | 6.0 | | 27.1 | 21.0 |
| 16.3 | 7.2 | | 28.0 | 3.2 |
| 17.2 | 13.7 | | 28.9 | 6.5 |
| 17.5 | 9.1 | | 29.6 | 4.6 |
| 18.2 | 57.9 | | 30.3 | 14.2 |
| 18.4 | 53.5 | | 32.4 | 3.4 |
| 18.9 | 14.2 | | 33.4 | 7.6 |
| 19.4 | 20.3 | | | |

The diffraction peaks at 3.7, 7.3, 11.0, 14.7, 18.2, 18.4, 19.4, 22.1, 23.6, 23.8, 25.9, 26.1 and 27.1±0.2 degrees 2θ are characteristic of this particular form.

The aripiprazole maleate of the present invention also can be characterized by a unique infra-red spectrum, e.g., as depicted in Figure 14, and by characteristic DSC and TGA curves, e.g., as depicted in Figures 15 and 16, respectively. According to the DSC curve, aripiprazole maleate is anhydrous, wherein the peak at about 161 °C probably belongs to an impurity; however, melting is not observed at this temperature.

The present invention further provides a crystalline solid comprising aripiprazole malonate, which can be characterized by a unique powder X-ray diffraction pattern, e.g., as depicted in Table 7 and Figure 17.

**Table 7 - Aripiprazole malonate - Powder X-ray diffraction peak positions and intensities.**

| 2θ degrees | I/I₀ | | 2θ degrees | I/I₀ |
|---|---|---|---|---|
| 7.7 | 31.0 | | 22.5 | 23.8 |
| 9.5 | 4.0 | | 23.4 | 46.2 |
| 10.3 | 22.1 | | 23.9 | 75.7 |
| 11.7 | 4.2 | | 24.5 | 89.7 |
| 12.3 | 12.4 | | 25.8 | 50.2 |
| 14.1 | 7.6 | | 27.4 | 25.8 |
| 14.7 | 5.5 | | 27.9 | 17.3 |
| 15.1 | 14.6 | | 28.1 | 16.5 |
| 15.5 | 34.6 | | 29.1 | 24.2 |
| 16.0 | 17.2 | | 30.0 | 8.0 |
| 16.7 | 91.0 | | 30.5 | 6.8 |
| 17.0 | 50.1 | | 31.1 | 8.4 |
| 17.9 | 100.0 | | 31.8 | 5.8 |
| 19.5 | 42.5 | | 32.3 | 12.4 |
| 20.1 | 15.5 | | 32.7 | 6.2 |
| 20.7 | 26.0 | | 33.8 | 4.6 |
| 21.5 | 26.5 | | 34.3 | 4.9 |
| 21.8 | 35.2 | | 34.6 | 5.3 |

The diffraction peaks at 7.7, 10.3, 15.5, 16.7, 17.0, 17.9, 19.5, 20.7, 21.5, 21.8, 22.5, 23.4, 23.9, 24.5, 25.8, 27.4 and 29.1 ± 0.2 degrees 2θ are characteristic of this particular form.

The aripiprazole malonate of the present invention also can be characterized by a unique infra-red spectrum, e.g., as depicted in Figure 18, wherein the band at 890 cm⁻¹ is characteristic of this form.

The aripiprazole malonate of the present invention also can be characterized by characteristic DSC and TGA curves, e.g., as depicted in Figures 19 and 20, respectively. According to the DSC curve, the peak at about 125°C probably is associated with melting and additional weight charge at higher temperature probably coincides with decomposition.

The present invention still further provides a crystalline solid comprising aripiprazole fumarate, which can be characterized by a unique powder X-ray diffraction pattern, e.g., as depicted in Table 8 and Figure 21.

**Table 8 - Aripiprazole fumarate - Powder X-ray diffraction peak positions and intensities.**

| 2θ degrees | I/I₀ | | 2θ degrees | I/I₀ |
|---|---|---|---|---|
| 6.6 | 20.5 | | 21.1 | 16.3 |
| 8.8 | 27.7 | | 21.8 | 92.8 |
| 11.1 | 43.9 | | 23.0 | 28.8 |
| 12.0 | 26.0 | | 23.4 | 25.0 |
| 13.2 | 7.3 | | 24.6 | 65.2 |
| 13.9 | 3.1 | | 25.8 | 25.4 |
| 15.6 | 38.1 | | 26.9 | 31.0 |
| 16.3 | 17.4 | | 28.0 | 11.2 |
| 17.4 | 23.6 | | 28.7 | 11.7 |
| 17.8 | 100.0 | | 29.6 | 5.9 |
| 18.9 | 11.5 | | 32.0 | 8.6 |
| 19.7 | 71.9 | | 33.7 | 4.9 |
| 20.1 | 33.1 | | | |

The diffraction peaks at 6.6, 8.8, 11.1,12.0,15.6,17.4, 17.8, 19.7, 20.1, 21.8, 23.0, 23.4, 24.6, 25.8 and 26.9 ± 0.2 degrees 29 are characteristic of this particular form.

The aripiprazole fumarate of the present invention also can be characterized by a unique infra-red spectrum, e.g., as depicted in Figure 22. The bands at 1680 cm⁻¹, 1053 cm⁻¹, and 713 cm⁻¹ are characteristic of this form. The aripiprazole fumarate of the present invention also can be characterized by characteristic DSC and TGA curves, e.g., as depicted in Figures 23 and 24, respectively. The present invention further provides a process for preparing crystalline aripiprazole fumarate, which, when obtained by crystallization from ethyl acetate, comprises partially amorphous material.

The present invention still further provides a crystalline solid comprising aripiprazole L-tartrate, which is characterized by a unique powder X-ray diffraction pattern, e.g., as depicted in Table 9 and Figure 25.

**Table 9 - Aripiprazole L-tartrate - Powder X-ray diffraction peak positions and intensities.**

| 2θ degrees | I/I₀ | | 2θ degrees | I/I₀ |
|---|---|---|---|---|
| 3.7 | 6.6 | | 23.4 | 82.3 |
| 11.0 | 12.3 | | 24.0 | 16.5 |
| 11.4 | 16.0 | | 25.0 | 59.9 |
| 12.2 | 6.4 | | 25.9 | 33.6 |
| 13.2 | 5.1 | | 26.5 | 16.4 |
| 15.3 | 56.1 | | 27.1 | 17.0 |
| 15.8 | 100.0 | | 28.3 | 19.0 |
| 16.3 | 33.3 | | 29.2 | 7.1 |
| 16.9 | 50.9 | | 29.9 | 5.5 |
| 17.6 | 52.6 | | 30.3 | 5.5 |
| 18.6 | 37.7 | | 31.5 | 5.3 |
| 19.7 | 7.2 | | 33.5 | 6.7 |
| 20.4 | 9.8 | | 34.2 | 7.8 |
| 21.8 | 63.14 | | | |

The diffraction peaks at 15.3, 15.8, 16.3, 16.9, 17.6, 18.6, 21.8, 23.4, 25.0 and 25.9 ± 0.2 degrees 2θ are characteristic of this form.

The aripiprazole L-tartrate of the present invention also can be characterized by a unique infra-red spectrum, e.g., as depicted in Figure 26. The bands at 1716 cm⁻¹, 1671 cm⁻¹, 1119 cm⁻¹ and 1072 cm⁻¹ are characteristic of this form. The aripiprazole L-tartrate of the present invention also can be characterized by characteristic DSC and TGA curves, e.g., as depicted in Figures 27 and 28, respectively.

In another embodiment, the present invention provides a crystalline solid comprising aripiprazole L-malate, which can be characterized by a unique powder X-ray diffraction pattern, e.g., as depicted in Table 10 and Figure 29.

**Table 10 - Aripiprazole L-malate - Powder X-ray diffraction peak positions and intensities.**

| 2θ degrees | I/I₀ | | 2θ degrees | I/I₀ |
|---|---|---|---|---|
| 3.7 | 6.9 | | 21.5 | 14.7 |
| 7.6 | 4.8 | | 22.4 | 100.0 |
| 11.5 | 10.7 | | 23.9 | 14.2 |
| 12.4 | 11.1 | | 25.3 | 47.0 |
| 13.2 | 4.0 | | 26.7 | 13.0 |
| 15.4 | 21.5 | | 27.3 | 8.7 |
| 16.2 | 46.0 | | 28.4 | 7.2 |
| 17.3 | 99.7 | | 29.1 | 15.2 |
| 18.4 | 23.7 | | 29.4 | 14.9 |
| 19.0 | 41.7 | | 31.2 | 9.8 |
| 20.4 | 7.4 | | 32.5 | 5.8 |

The diffraction peaks at 15.4, 16.2, 17.3, 18.4, 19.0, 22.4 and 25.3 ± 0.2 degrees 2θ are characteristic of this particular form.

The aripiprazole L-malate also can be characterized by a unique infra-red spectrum, e.g., as depicted in Figure 30. The band at 847 cm⁻¹ is characteristic of this form. The aripiprazole L-malate also can be characterized by characteristic DSC and TGA curves, e.g., as depicted in Figures 31 and 32, respectively.

In yet another embodiment, the present invention provides a crystalline solid comprising aripiprazole citrate, which can be characterized by a unique powder X-ray diffraction pattern, e.g., as depicted in Table 11 and Figure 33.

**Table 11 - Aripiprazole citrate - Powder X-ray diffraction peak positions and intensities.**

| 2θ degrees | I/I₀ | | 2θ degrees | I/I₀ |
|---|---|---|---|---|
| 7.0 | 4.6 | | 20.9 | 9.1 |
| 10.5 | 5.2 | | 22.2 | 77.7 |
| 11.6 | 12.4 | | 23.2 | 51.3 |
| 12.9 | 17.7 | | 25.1 | 73.6 |
| 14.0 | 48.0 | | 26.7 | 21.5 |
| 16.4 | 100.0 | | 27.7 | 15.2 |
| 17.2 | 65.7 | | 28.7 | 11.6 |
| 17.9 | 24.4 | | 29.5 | 12.7 |
| 19.4 | 8.4 | | 32.1 | 5.5 |
| 20.0 | 15.7 | | 32.7 | 6.8 |

The diffraction peaks at 14.0, 16.4, 17.2, 17.9, 22.2, 23.2, 25.1 and 26.7 ± 0.2 degrees 2θ are characteristic of this particular form.

The aripiprazole citrate also can be characterized by a unique infra-red spectrum, e.g., as depicted in Figure 34. The bands at 1725 cm⁻¹, 1169 cm⁻¹ and 1195 cm⁻¹ are characteristic of this form. The aripiprazole citrate also can be characterized by DSC and TGA curves, e.g., as depicted in Figures 35 and 36, respectively.

The aripiprazole salts of the present invention can prepared by any suitable process, e.g., by suspending aripiprazole, obtained by any suitable method known in the art, in an organic solvent, e.g., a C₁-C₄ alcohol (e.g., ethanol) or a water-immiscible solvent (e.g., ethyl acetate), optionally with heating (e.g., to reflux temperature), adding a solution of an organic or a mineral acid in, e.g., a C₁-C₄ alcohol (e.g., ethanol) and crystallizing the thus obtained salt, e.g., by cooling, and isolating the crystals by any suitable method, e.g., by filtration.

The present invention further provides a process for preparing aripiprazole salts, which process preferably comprises:
suspending aripiprazole in an organic solvent;
heating the mixture to elevated temperature, preferably to reflux to form a solution;
adding a solution of an organic or a mineral acid in an organic solvent;
allowing the mixture to cool sufficiently to produce crystals of an aripiprazole salt;
collecting the obtained crystals, preferably by filtration; and
optionally washing and drying the crystals, e.g., wherein the drying is optionally performed under reduced pressure.

The organic solvent used in accordance with the process of the present invention can include one or more C₁-C₄ alcohols, preferably ethanol, and one or more water-immiscible solvents such as, e.g., methyl acetate, ethyl acetate, n-propyl acetate, isopropyl acetate, n-butyl acetate or isobutyl acetate, or a combination thereof.

In accordance with the present invention, novel aripiprazole salts have been obtained in high purities. The conversion of aripiprazole base to the new salts can serve as a convenient process for the purification of aripiprazole, which may be optionally converted to aripiprazole hydrochloride.

Thus, for example, when crude aripiprazole base, having a purity of about 98% (by HPLC), e.g., as described in Examples 1 and 2 of the present application, is treated with maleic acid, aripiprazole maleate can be obtained and optionally crystallized (e.g., from ethanol). The aripiprazole salt thus obtained can be converted to the base, having a high purity, e.g., about 99.3% as described in Example 18.

The present invention also provides a process for preparing highly pure aripiprazole base in high yield from one or more of the crystalline aripiprazole salts of the present invention. In one embodiment, the process of the present invention for obtaining highly pure aripiprazole base comprises:
providing a two-phase mixture of an aripiprazole salt of the present invention, an organic solvent and water;
adding a base, optionally with stirring and heating to an elevated temperature;
separating the layers;
adding water to the organic phase, optionally with stirring and heating;
distilling off at least a portion of one or more of the solvents; and
adding a second organic solvent and cooling to precipitate the aripiprazole; and
optionally isolating the aripirprazole, e.g., by filtration.

In accordance with the present invention, exemplary organic solvents that can be used in the two-phase solvent mixture include, but are not limited to, toluene, xylenes, ethylbenzene, pentane, or a mixture thereof. A preferred organic solvent that can be used in the two-phase solvent mixture is toluene. Exemplary solvents that can be used as the second organic solvent include, but are not limited to, C₁-C₄ alcohols, e.g., ethanol, or a water-immiscible solvent such as, e.g., methyl acetate, ethyl acetate, n-propyl acetate, isopropyl acetate, n-butyl acetate, isobutyl acetate or a combination thereof. A preferred solvent that can be used as the second organic solvent is ethanol. Exemplary bases can include, but are not limited to, lithium hydroxide, sodium hydroxide, potassium hydroxide, sodium carbonate, potassium carbonate, or a combination thereof. A preferred base is sodium hydroxide.

Although, the following examples illustrate the practice of the present invention in some of its embodiments, the examples should not be construed as limiting the scope of the invention. Other embodiments will be apparent to one skilled in the art from consideration of the specification and examples. It is intended that the specification, including the examples, is considered exemplary only without limiting the scope and spirit of the invention.

The following apply with respect to the powder X-ray diffraction studies:
two-theta min.: 3.00; two-theta max.: 35.00; step size: 0.05; count time (sec): 50; receiving slit (mm): 0.2; scattering slit (mm): 1; KV: 40. The following apply with respect to the infrared spectra: number of sample scans: 16; number of background scans: 16; resolution: 4.000; sample gain: 1.0 - 2.0; mirror velocity: 0.6329; aperture: 100.00. DSC scans were performed on a DSC Q1000 V8.2 Build 268 differential scanning calorimeter (ramp: 10.00 °C/min. to 300.00 °C). The following apply with respect to the thermogravimetric analyses: instrument: TGA Q500 V6.2 Build 187; cell constant: 1.00000; temperature calibration: 25.50,25.50,153.56, 154.16; ramp: 10.00 °C/min. to 300.00 °C.

### EXAMPLE 1

This example describes the preparation of aripiprazole by reaction of 1-(2,3-dichlorophenyl)piperazine monohydrochloride with 7-(4-chlorobutoxy)-3,4-dihydro-2(1H)-quinolinone in the presence of phase transfer catalyst and potassium carbonate in a bi-phasic mixture containing toluene and water.

A reaction vessel was charged with 7-(4-chlorobutoxy)-3,4-dihydro-2(1H)-quinolinone [15.3 g, 0.064 mole], 1-(2,3-dichlorophenyl)piperazine mono hydrochloride (17.8 g, 0.0665 mole), potassium carbonate (9.2 g , 0.0667 mole), tetrabutylammonium bromide (1.8 g), toluene (230 ml) and water (92 ml). The mixture was heated under reflux for 13 hours. Then, the reaction mixture was cooled to about 65 °C and toluene was added (230 ml) and stirring was maintained for 15 minutes. The phases were separated and the aqueous phase was collected (about 96 ml). Water (77 ml) was added to the organic phase and the mixture was stirred at about 65 °C for 15 minutes. The layers were separated and toluene was distilled out (about 184 ml). Ethanol was added (230 ml) in portions at 65 °C to afford a solution. The solution was cooled to about 25 °C and stirred at that temperature for one hour. Then, the solution was cooled to about 5 °C and stirred at that temperature for one hour. The precipitate was collected by filtration and washed with ethanol to obtain a wet solid, which was dried at 60 °C to afford dry crude aripiprazole (17.6 grams, 65% yield), having a purity of 98%.

### EXAMPLE 2

This example describes the preparation of aripiprazole maleate by crystallization from ethanol.

In a three necked round bottom flask equipped with a reflux condenser, a thermometer and a magnetic stirrer, aripiprazole (obtained by any method know in the art, e.g., according to example 1) (3 gram) was suspended in absolute ethanol (30 ml). The suspension was heated to reflux to form a solution. Then, a solution of 1.85M maleic acid in ethanol (0.86 grams of maleic acid in 4 ml ethanol) was added while maintaining the reflux temperature during few minutes. The mixture was left to cool to room temperature and stirred at room temperature for about an hour. Then, the mixture was cooled to about 5°C and stirred at that temperature for about an hour. The resulting crystals were filtered, washed with cold ethanol (2 ml) and dried under reduced pressure to afford 3.6 gram of aripiprazole maleate in 96% yield.

### EXAMPLES 3-9

This example describes the preparation of other aripiprazole salts by crystallization from ethanol.

Preparation of other aripiprazole salts by crystallization from ethanol was carried out in the same manner as described in example 2, using different organic acids. The results are summarized in Table 12.

**Table 12 - Preparation of aripiprazole salts by crystallization from ethanol using various organic acids.**

| Example No. | Organic acid | Yield |
|---|---|---|
| 3 | oxalic acid | 78% |
| 4 | benzoic acid | 63% |
| 5 | malonic acid | 82% |
| 6 | fumaric acid | 78% |
| 7 | L-tartaric acid | 97% |
| 8 | L-malic acid | 88% |
| 9 | citric acid | 89% |

### EXAMPLE 10

This example describes the preparation of aripiprazole maleate by crystallization from ethyl acetate.

In a three necked round bottom flask equipped with a reflux condenser, a thermometer and a magnetic stirrer, aripiprazole (obtained by any method know in the art) (3 gram) was suspended in ethyl acetate (30 ml). The suspension was heated to reflux to form a solution. Then, a solution of 1.85M maleic acid in ethanol (0.86 grams of maleic acid in 4 ml ethyl acetate) was added while maintaining the reflux temperature during few minutes. The mixture was left to cool to room temperature and stirred at room temperature for about an hour. Then, the mixture was cooled to about 5°C and stirred at that temperature for about an hour. The resulting crystals were filtered, washed with cold ethyl acetate (2 ml) and dried under reduced pressure to afford 3.3 gram of aripiprazole maleate in 88% yield.

### EXAMPLES 11-17

This example describes the preparation of other aripiprazole salts by crystallization from ethyl acetate.

Preparation of other aripiprazole salts by crystallization from ethanol was carried out in the same manner as described in example 9, using different organic acids. The results are summarized in table 13.

**Table 13 - Preparation of other aripiprazole salts by crystallization from ethyl acetate using different acids.**

| Example No. | Organic acid | Yield |
|---|---|---|
| 11 | oxalic acid | 89% |
| 12 | benzoic acid | 41 % |
| 13 | malonic acid | 84% |
| 14 | fumaric acid | 90% |
| 15 | L-tartaric acid | 39% |
| 16 | L-malic acid | 92% |
| 17 | citric acid | 94% |

### EXAMPLE 18

This example describes the preparation of aripiprazole base from aripiprazole maleate.

A reaction vessel was charged with aripiprazole maleate (11.0 g), toluene (94 ml) and water (37 ml). The mixture was heated to 65°C under stirring, and 47% sodium hydroxide (2.5 ml) was added in portions. The pH was checked and a value of about 11 was obtained. Stirring was continued at 65°C for 15 minutes and the layers were separated. Water (32 ml) was added to the toluene layer and stirring of the mixture was maintained at 80°C for 15 minutes. The layers were separated and water was distilled out by performing an azeotropic distillation. The distillation was continued until the toluene distillate was clear. The majority of the toluene was distilled out at atmospheric pressure and toluene (94 ml) was collected. The mixture was cooled to 100°C and absolute ethanol (33 ml) was added to afford a solution. The hot solution was filtered and the hot filtrate was transferred to another reaction vessel. The mixture was cooled to 25°C to afford crystallization of the product from the solution. Stirring was maintained for 1 hour at 25°C, then for 1 hour at 5°C. A cake was obtained by filtration and washed with cold absolute ethanol. The cake was dried at 60°C in vacuum to afford 8.0 g of pure aripiprazole base in 91 % yield, having a purity of 99.3%.

The preparation of aripiprazole base from different aripiprazole salts, in the same manner as described in this example, is summarized in Table 14.

**Table 14 - Preparation of aripiprazole from different aripiprazole salts.**

| Example No. | Aripiprazole Salt | Yield of the obtained aripiprazole base | Purity of the obtained aripiprazole base |
|---|---|---|---|
| 19 | aripiprazole L-tartrate | 84% | 99.4% |
| 20 | aripiprazole malonate | 76% | 99.0% |

The preparation of aripiprazole base from different aripiprazole salts, in the same manner as described in this example, is summarized in Table 14. All references, including publications, patent applications, and patents, cited herein are hereby incorporated by reference to the same extent as if each reference were individually and specifically indicated to be incorporated by reference and were set forth in its entirety herein.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

## Claims

1. A crystalline carboxylic acid salt of 7-(4-(4-(2,3-dichlorophenyl)-1-piperazinyl)-butoxy)-3,4-dihydro-2(1H)-quinolinone (aripiprazole).

2. The crystalline salt of claim 1, comprising aripiprazole oxalate, aripiprazole benzoate form I, aripiprazole benzoate form II, aripiprazole maleate, aripiprazole malonate, aripiprazole fumarate, aripiprazole L-tartrate, aripiprazole L-malate, aripiprazole citrate, or a combination thereof.

3. The crystalline salt of claim 1, comprising aripiprazole oxalate, which exhibits a powder X-ray diffraction pattern having strong diffraction peaks at 11.9, 16.6, 17.4, 18.1, 21.2, 22.8, 24.1 and 25.3 ± 0.2 degrees 2θ.

4. The crystalline salt of claim 1, comprising aripiprazole oxalate, which exhibits an IR spectrum having a characteristic band at 1170 cm⁻¹ and a doublet at 768 cm⁻¹ and 779 cm⁻¹.

5. The crystalline salt of claim 1, comprising aripiprazole benzoate form I, which exhibits a powder X-ray diffraction pattern with strong diffraction peaks at 8.8, 11.7, 15.8, 16.4, 17.8, 18.7, 20.3, 23.4 and 25.0 ± 0.2 degrees 2θ.

6. The crystalline salt of claim 1, comprising aripiprazole benzoate form I, which exhibits an infra-red spectrum with bands at 1682 cm⁻¹, 1310 cm⁻¹, 1293 cm⁻¹,1274 cm⁻¹ and 862 cm⁻¹.

7. The crystalline salt of claim 1, comprising aripiprazole benzoate form I, which exhibits a DSC curve with a characteristic transition at 132°C.

8. The crystalline salt of claim 1, comprising aripiprazole benzoate form II, which exhibits a powder X-ray diffraction pattern with peaks at 17.4, 18.1, 19.6, 23.2 and 24.4 ± 0.2 degrees 2θ.

9. The crystalline salt of claim 1, comprising aripiprazole benzoate form II, which exhibits an infra-red spectrum with a characteristic band at 1676 cm⁻¹.

10. The crystalline salt of claim 1, comprising aripiprazole benzoate form II, which exhibit DSC and TGA curves consistent with an ethanol solvate containing about 2% of ethanol.

11. The crystalline salt of claim 1, comprising aripiprazole maleate, which exhibits a powder X-ray diffraction pattern with peaks at 3.7, 7.3, 11.0, 14.7, 18.2, 18.4, 19.4, 22.1, 23.6, 23.8, 25.9, 26.1 and 27.1± 0.2 degrees 2θ.

12. The crystalline salt of claim 1, comprising aripiprazole maleate, which exhibits a DSC curve consistent with anhydrous aripiprazole maleate.

13. The crystalline salt of claim 1, comprising aripiprazole malonate, which exhibits a powder X-ray diffraction pattern with peaks at 7.7, 10.3, 15.5, 16.7, 17.0, 17.9, 19.5, 20.7, 21.5, 21.8, 22.5, 23.4, 23.9, 24.5, 25.8, 27.4 and 29.1 ± 0.2 degrees 2θ.

14. The crystalline salt of claim 1, comprising aripiprazole malonate, which exhibits an infra-red spectrum with a characteristic band at 890 cm⁻¹.

15. The crystalline salt of claim 1, comprising aripiprazole malonate, which exhibits a DSC curve with a peak at about 125°C consistent with melting.

16. The crystalline salt of claim 1, comprising aripiprazole fumarate, which exhibits a powder X-ray diffraction pattern with peaks at 6.6, 8.8, 11.1, 12.0, 15.6, 17.4, 17.8, 19.7, 20.1, 21.8, 23.0, 23.4, 24.6, 25.8 and 26.9 2 ± 0.2 degrees 2θ.

17. The crystalline salt of claim 1, comprising aripiprazole fumarate, which exhibits an infra-red spectrum with characteristic bands at 1680 cm⁻¹, 1053 cm⁻¹ and 713 cm⁻¹.

18. The crystalline salt of claim 1, comprising aripiprazole L-tartrate, which exhibits a powder X-ray diffraction pattern with peaks at 15.3, 15.8, 16.3, 16.9, 17.6, 18.6, 21.8, 23.4, 25.0 and 25.9 ± 0.2 degrees 2θ.

19. The crystalline salt of claim 1, comprising aripiprazole L-tartrate, which exhibits an infra-red spectrum with characteristic absorption bands at 1716 cm⁻¹, 1671 cm⁻¹, 1119 cm⁻¹ and 1072 cm⁻¹.

20. The crystalline salt of claim 1, comprising aripiprazole L-malate, which exhibits a powder X-ray diffraction pattern with peaks at 15.4, 16.2, 17.3, 18.4, 19.0, 22.4 and 25.3 ± 0.2 degrees 2θ.

21. The crystalline salt of claim 1, comprising aripiprazole L-malate, which exhibits an infra-red spectrum with a characteristic absorption band at 847 cm⁻¹.

22. The crystalline salt of claim 1, comprising aripiprazole citrate, which exhibits a powder X-ray diffraction pattern with peaks at peaks at 14.0, 16.4, 17.2, 17.9, 22.2, 23.2, 25.1 and 26.7 ± 0.2 degrees 2θ.

23. The crystalline salt of claim 1, comprising aripiprazole citrate, which exhibits an infra-red spectrum with characteristic absorption bands at 1725 cm⁻¹, 1169 cm⁻¹, and 1195 cm⁻¹.

24. A crystalline solid comprising the aripiprazole salt of claim 1.

25. A process for preparing a salt of aripiprazole, the process comprising:
dissolving aripiprazole in an organic solvent, optionally by heating a suspension of aripiprazole in the solvent;
adding a solution of an acid in an organic solvent;
allowing the mixture to cool sufficiently to produce crystals of the aripiprazole salt;
isolating the crystals; and
optionally washing and drying the crystals, wherein the drying is optionally performed under reduced pressure.

26. The process of claim 25, wherein the organic solvent is a C₁-C₄ alcohol, methyl acetate, ethyl acetate, n-propyl acetate, isopropyl acetate, n-butyl acetate, isobutyl acetate, or a combination thereof.

27. The process of claim 26, wherein the organic solvent is ethanol, ethyl acetate or a combination thereof.

28. A process for producing aripiprazole, the process comprising:
providing a two-phase mixture comprising the aripiprazole salt of claim 1, an organic solvent and water;
adding a base, optionally with stirring and heating;
separating the layers and adding water to the organic phase, optionally with stirring and heating;
distilling off at least a portion of the solvents;
adding a second organic solvent and cooling to precipitate aripiprazole; and
optionally isolating the aripiprazole.

29. The process of claim 28, wherein the organic solvent in the two-phase solvent mixture comprises toluene, one or more xylenes, ethylbenzene, pentane, or a combination thereof.

30. The process of claim 29, wherein the organic solvent is toluene.

31. The process of claim 28, wherein the second organic solvent comprises a C₁-C₄ alcohol, methyl acetate, ethyl acetate, n-propyl acetate, isopropyl acetate, n-butyl acetate, isobutyl acetate, or a combination thereof.

32. The process of claim 31, wherein, the second organic solvent is ethanol.

33. The process of claim 28, wherein the base comprises lithium hydroxide, sodium hydroxide, potassium hydroxide, sodium carbonate, potassium carbonate or a combination thereof.

34. The process of claim 33, wherein the base is sodium hydroxide.
